# EUROPEAN PATENT APPLICATION

(11) **EP 0 841 156 A1**
(43) Date of publication of application: **13.05.1998**
(21) Application number: 96922261.1
(22) Date of filing: 09.07.1996
(51) Int. Cl.: B32B 5/26, A61F 13/15

(54) **POROUS COMPOSITE SHEET AND PROCESS FOR THE PRODUCTION THEREOF**

(30) Priority: 10.07.1995 JP 69819/95 U; 19.09.1995 JP 23959/95
(71) Applicant: JAPAN ABSORBENT TECHNOLOGY INSTITUTE, Chuo-ku, Tokyo 103 (JP)
(72) Inventor: SUZUKI, Migaku, 19-2, Ueki, Kamakura-shi, Kanagawa 247 (JP); FUKUI, Hiroaki, Kawaguchi-shi, Saitama 332 (JP)
(74) Representative: Klingseisen, Franz, Dipl.-Ing.
(86) International application number: PCT/JP96/01893
(87) International publication number: WO 97/02946

(57) **Abstract**

A sintered porous composite sheet, comprising an A/B-component layer in whic h A-component having easily fusible properties and B-component having relatively higher thermal stability as compared with A-component coexist in a sintered state. A-component can comprise a hydrophilic material, B-component can comprise a hyd rophobic material, and A-component and B-component can contain a common mater ial comprising an easily fusible material. The porous composite sheet has advantag es that it has moderate air- or moisture permeability and is excellent in drapabilit y; and is most suitable to a material for sanitary or medical wares.

## Description

### Technical Field

The present invention relates to a novel sintered porous composite sheet having multilayered structure, more particularly relates to a sintered porous composite sheet which has a proper air-permeability or moisture permeability and is excellent in drapability. This sintered porous composite sheet is useful as a top or back sheet for a sanitary article, or a high performance material for a medical application, etc., in which the permeability of said composite sheet to air or water vapor through the pores thereof, and the water-permeability thereof by a combination of a hydrophobic layer and a hydrophilic layer are utilized. Furthermore, the present invention also relates to a method of producing such sintered porous composite sheet.

### Background Art

Generally, sheet articles having a porous structure are classified into the following four groups according to the pore size:

| Pore Size(µm) | Materials | Performances |
|---|---|---|
| 0.01-0.1 | Gas-Barrier Film | Gas Permeability |
| 0.1-1.0 | Film Filter | Water-Vapor Permeability |
| 1.0-10.0 | Meltblown, Flash-spinning Web | Biobarrier |
| 10.0-100 | Conventional Nonwoven Fabric | Porous Articles |
| 100-1000 | Perforated Nonwoven Fabric | Perforated Articles |

Among these porous sheets, the ones having a pore size of about 10 µm or less are generally referred to as "microporous materials", which are produced according to a special techniques for forming films, such as an extracting method, a phase separation method or a method which comprises adding an inorganic powder in a high concentration and stretching in a biaxial direction; and are widely applied to special filters, air-permeable water-proof sports-wears and the like. These porous sheets are used as composites in which a nonwoven fabric and/or a woven fabric are used as support materials, because said porous sheets are hard in the form of a single substance, and have a problem in strength.

Furthermore, as another method, a method is considered which comprises first forming a porous sheet having a relatively large pore-size, and then hot-crimping into a film to increase the density thereof so as to provide a microporous structure. It would be in principle possible to obtain a microporous film by applying ideas for producing, for example, a filter (or a filter paper) as obtained by matting and pressing a refined wood-pulp fiber; a high-temperature and high-pressure pressed sheet of an urethane foam sponge; or a synthetic-paper-like sheet which is provided by pressing and hot-crimping a dry-formed sheet of flash-spinned fibrils of PE/PP or a wet-formed sheet of a synthetic pulp in their original condition, and further by selecting the conditions thereof.

Besides, Japanese Patent KOKAI (Laid-Open) No. 14023/89 discloses a method of producing a porous sheet by drawing a film which comprises a crystalline polyolefine resin, a rubber-like polymer and an inorganic filler; hot-crimping said oriented film into a mesh-like sheet; fixing said mesh-like sheet; and thermal contracting the same.

However, a porous sheet according to a conventional method as mentioned above is generally hard and easily turns to a fragile one in the form of a single body. Therefore, such a porous sheet is not satisfactory as a high performance material which is required for the application thereof to a sanitary article, a medical article, etc.

Furthermore, What is obtained according to a method disclosed in Japanese Patent KOKAI No. 14023/89 has a problem in the stability of the charaterictics thereof, because it is easily and directly affected by the ununiformity of the surface of a mesh-like sheet.

It is an object of the present invention to provide a high-performance porous sheet in which the defect of a porous sheet obtained according to a conventional method as mentioned above is dissolved, which is applicable to a sanitary article, a medical article, etc.

It is another object of the present invention to provide a method of producing such a porous sheet.

### Disclosure of the Invention

According to the present invention, a sintered porous composite sheet is provided, which is characterized by comprising an A/B-component layer in which A-component having easily fusible properties and B-component having higher thermal stability as compared with A-component are sintered together and coexist.

When a layer comprising A-component and a layer comprising B-component are sintered together so as to produce an A/B-component layer, such conditions that the compositions of part of said layer comprising A-component and/or said layer comprising B-component are left in their original condition are selected so as to form a sintered porous composite sheet having two-layers or three-layers structure, in which a layer which has easily fusible properties and consists of A-component only and/or a B-component layer which consists of relatively higher thermal stability are left.

Furthermore, according to the present invention, a method of producing a sintered porous composite sheet, characterized in that a first porous layer and a second porous layer are superposed, said first layer having easily fusible properties and comprising A-component, said second layer comprising B-component having relatively higher thermal stability as compared with A-component; and said first and second porous layers are thermal treated under pressure and under such temperature conditions that A-component is hot-molten while said B-component is stable so as to melt A-component and move the same into B-component, followed by cooling and solidifying the same, whereby three layer comprising an A-component layer which is molten and resolidified, an A/B-component layer, and a B-component layer having low thermal deformation are formed.

Moreover, according to the present invention, a composite sheet having water-permeability, characterized in that said composite sheet comprises a first layer comprising a hydrophilic material and a second layer which is positioned so as to be adjacent to said first layer;
said first layer and said second layer contain a common component comprising an easily fusible material; and
said first layer and said second layer are bonded to each other at sintered sites which are formed by mutually sintering said common component which is contained in both layers,
whereby said composite sheet has water-permeability at said sintered sites.

The sintered sites may be formed substantially all over the surface of said composite sheet, or may be formed in any pattern merely at part of the surface.

The common material which is contained in the first ans second layers are preferably a fiber material having easily fusible properties. Furthermore, bicomponent fibers in which an easily fusible component is used as a sheath and a component having higher thermal stability as compared with the sheath is used as a core, can be advantageously used as the common material.

Furthermore, according to the present invention, an absorbent product comprising a liquid-impermeable outer-sheet and inner-sheet on the opposite side of a body, and an absorbent which is positioned between said outer sheet and said inner sheet, wherein said inner-sheet comprises the water-permeable composite sheet as mentioned above is provided.

The fundamental ideas of the present invention are the application of the principle that a preformed porous layer is hot-pressed so as to form a porous sheet, and is entirely different from the invention as disclosed in Japanese Patent KOKAI (Laid-Open) No. 14023/89 in applied means and mechanism.

Namely, according to the present invention, a structure is employed, in which two porous layers, each of which has a porous structure and comprises a raw material having different fusible properties, are combined so as to infiltrate at least part of a low fusible layer, i.e. a porous layer which has easily fusible properties into voids of a porous layer which has higher thermal stability, in a melting state; and are bonded to each other by pressing for joining, filling, and cooling for fixing.

Accordingly, a sintered porous composite sheet which is porous in an extremely large range and has any properties is obtained by selecting materials which two porous layers comprise and by selecting the conditions of the sintering consequent upon melting, pressing for joining, and filling.

Here one example will be cited for explanation. The following Table 1 takes some fiber materials as examples, and enumerates part of the optimum uses of functional composite sheets which are obtained depending on the differences of the finances, surface-activities, etc. of used constitutive-fibers.

**Table 1**

| Materials Constituting Porous First Layer | Materials Constituting Porous First Layer | Functional Material Obtained by Sintering |
|---|---|---|
| Up to 0.01d PP/PE Microfibrils | Hydrophobic, Water-repellent | Waterproof Material Having Air-Permeability and Moisture-Permeability |
| Up to 0.1d PP/PE Extra Fine Fibers | Cotton-Spunlace Nonwoven Fabric | Surgical gown Having Biobarrier Property |
| Up to 1d PP Spunbond Nonwoven Fabric | PVA Sponge | Dustproof Controlling Material |
| PE/PET-Bicomponent-Spunbond Nonwoven Fabric | Thermalbond Nonwoven Fabric containing Of Absorber Product Hydrophilicated PE/PET-Bicomponent Fibers | Surface Material |

Fundamental requirements for obtaining a sintered porous composite sheet having such functions will be considered as follows.

The fundamental requirements of the present invention lie in that both two layers to be bonded are porous; and the first layer can maintain a microporous structure owing to the porosity thereof at the binding sites said two layers even after fusible, while A-component of the first layer in a melting state infiltrates into a large number of voids of the second layer so that the voids are filled with A-component, whereby what is called sintered-form portions of both A-component and B-component are formed.

In order to maintain dimensional stability without losing the porous structure of B-component and without causing thermal contraction, etc. when A-component is heated to a sintering state, if the heat stability of A-component and B-component are represented by, for example, the difference between melting points thereof, or the difference between thermal decomposition temperatures thereof, the difference between those of A-component and B-component is desirably 30O°C or more, more desirably 50O°C or more.

As easily fusible materials which are A-component and satisfactory for such conditions, for example, polymeric materials such as PE, PP, PET and derivatives thereof, SEBS, SIS, SEPS; and the combinations thereof are enumerated.

On the other hand, as slightly fusible or non fusible materials which is suitable for A-component, cellulose; polyurethane; P.V.A.; polyphenol; polyacrylonitrile; polymeric materials such as polyester or nylon which has a relatively higher melting temperature in combination with A-component; polymeric materials such as derivatives thereof, and the combinations thereof are enumerated.

In the present specification, the term 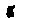 porous material" means a material having an apparent specific gravity of 0.2g/cm³ or less, preferably 0.1g/cm³ or less. However, as it may become difficult to hot-melt and compress a material having an apparent specific gravity of 0.02g/cm³ or less, it is necessary to pay attention.

As a preferable form of the first porous layer, for example, a meltblown nonwoven fabric, a wet-formed sheet of a synthetic pulp, a foam extruded net, molten extruded high-fibrillated net, a spunbond nonwoven fabric, a nonwoven fabric by card web method, or a sheet material which is obtained by any combination thereof is enumerated.

On the other hand, as the second porous layer, a foam sheet; a wood-pulp sheet which is formed according to air-laid method; a fiber mat; tissue paper; rayon or cotton-web; a cellulose web which was made into a CMC; a polyacrylonitrile-fiber web which was partially hydrolyzed; a synthetic-fiber web such as polyester or nylon, which has a relatively higher melt-concentration in combination with A-component; and a mixed material thereof are enumerated.

A combination of said first layer and said second layer is selected and used according to the purpose thereof, and in particular, a combination of a first layer comprising a hydrophobic material and a second layer comprising a hydrophilic material is most suitable to a raw material particularly for a sanitary article and a medical article. Namely, a composite sheet having common binding sites has properties remarkably excellent in water-permeability, wherein said binding sites are formed by mutually sintering a common A-component comprising an easily fusible material which is contained in each of said first and second layers.

In a sintered porous composite sheet of the present invention, heating and pressing are required so as to sinter a first and second porous sheets, while according to the grades of the sintering, the following three modes can be employed:
(a) a mode that a first porous layer is made into a film so as to form three layers comprising an A-component layer, A/B-component mixed layer and a B-component layer; and A-component is distributed so as to be divided into a layer comprising a A-component only which is left as it is, and an A/B-component layer in which part of A-component is shifted into the B-component layer, while the rate of A-component to shift into the B-component layer is preferably 50% or more by weight of A-component in the initial A-component layer, more preferably 70% or more so as to form the A/B-component layer;
(b) a mode that almost all of A-component in a first layer is shifted into the B-component layer as a second layer so as to form a sintered composite sheet comprising an A/B-component layer and the B-component layer; and
(c) a mode that almost all of an A-component layer in the first layer and almost all of a B-component layer in the first layer are mixed with each other so as to form a sintered composite sheet comprising an A/B-component layer only.

An important matter in the present invention is that as a matter in common to any modes of the above-mentioned (a), (b) and (c), A/B-component layer in which A-component and B-component coexist lies.

Means for forming sintered structure in which A/B-component coexist are divided broadly into a method by which the A-component layer and the B-component layer are superposed on each other, and then the A-component layer is hot-molten so as to be infiltrated and pressed into the B-component layer; and a method by which first the A/B-component layer is formed, and in that state thereof the A-component layer is sintered and pressed for joining.

### Brief Description of Drawings

Figure 1 is a schematic diagram of a process in which a sintered porous composite sheet of the present invention is produced.
Figure 2 is a schematic diagram which illustrates the transition of two layers in a process in which a sintered porous composite sheet of the present invention is produced.
Figure 3A is a schematic diagram which illustrates a process in which a sintered porous composite sheet of the present invention from different materials is produced.
Figure 3B is a schematic Nonwoven Fabric diagram which illustrates a process in which bicomponent fibers are used so as to produce a sintered porous composite sheet of the present invention.
Figure 4 is a schematic diagram which illustrates a method of testing bio-barrier properties of a sintered porous composite sheet of the present invention.
Figures 5(a) to 5(d) are schematic diagrams of a process in which a sintered porous composite sheet of the present invention is produced.
Figures 6(a) to 6(c) are plan views which illustrate a first embodiment of a sintered porous composite sheet of the present invention.
Figure 7 is a plan view which illustrates a second embodiment of the present invention.
Figure 8 is a partial drawing of a longitudinal section of Figure 7.
Figure 9 is a plan view which illustrates a third embodiment of the present invention.
Figure 10 is a partial drawing of a longitudinal section of Figure 9.
Figure 11 is a plan view which illustrates a fourth embodiment of the present invention.
Figure 12 is a perspective drawing of elements on larger scale of Figure 11.
Figure 13 is a longitudinal section which illustrates a fifth embodiment of the present invention.
Figure 14 is a perspective drawing of elements on larger scale of Fig. 13.
Figure 15 is a sectional view of elements of an apparatus for producing a sintered porous composite sheet as shown in Figure 14.
Figure 16 is a schematic diagram which illustrates a change of states before and after water-absorption of a sintered porous composite sheet as shown in Figure 13.
Figure 17 is a longitudinal section which illustrates a sixth embodiment of the present invention.
Figure 18 is a longitudinal section which illustrates a seventh embodiment of the present invention.
Figure 19 is a plan view which illustrates an eighth embodiment of the present invention.
Figure 20 is a longitudinal section which illustrates a ninth embodiment of the present invention.

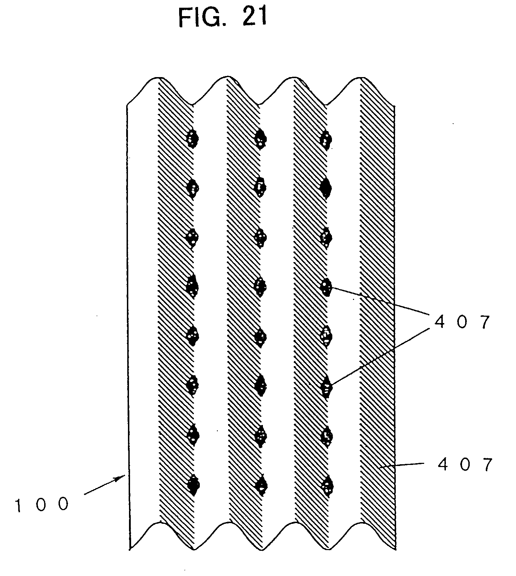

Figure 21 is a longitudinal section which illustrates a tenth embodiment of the present invention.
Figure 22 is a longitudinal section of elements of a water-permeable composite sheet of the embodiment of Figure 11.
Figure 23 is a longitudinal section of elements of a water-permeable composite sheet of the embodiment of Figure 12.
Figure 24 is a longitudinal section of elements of a water-permeable composite sheet of the embodiment of Figure 13.
Figure 25 is a longitudinal section of elements of a water-permeable composite sheet of the embodiment of Figure 14.
Figure 26 is a longitudinal section of elements of a water-permeable composite sheet of the embodiment of Figure 15.
Figure 27 is a perspective drawing of a product in which a sintered porous composite sheet of the present invention is used.
Figure 28 is a longitudinal section of Figure 22.
Figure 29 is a longitudinal section of a product in which a sintered porous composite sheet of the present invention is used.
Figure 30 is a schematic diagram which illustrates an embodiment of Figure 16.
Figure 31 is a schematic diagram which illustrates an embodiment of Figure 17.
Figure 32 is a plan view of a non-returnable diaper in which a water-permeable composite sheet of the present invention is used.
Figure 33 is a schematic longitudinal section at line Y-Y of the diaper shown in Figure 32.

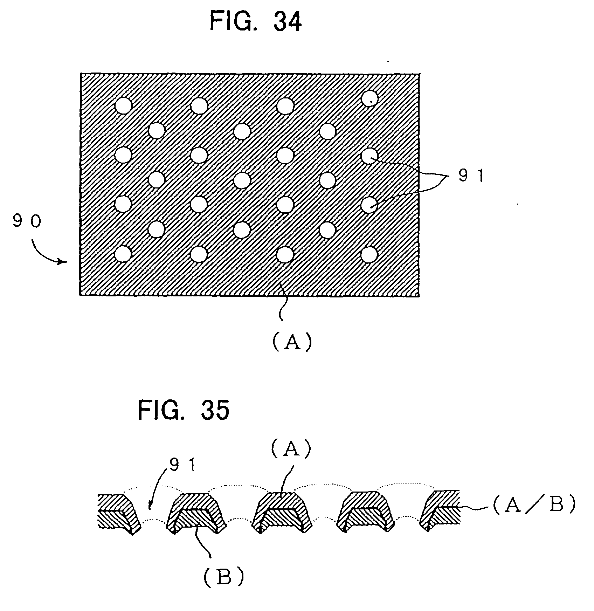

Figure 34 is a longitudinal section of elements of a water-permeable composite sheet of an eighteenth embodiment of the present invention.
Figure 35 is a longitudinal section of elements of a water-permeable composite sheet of Figure 6.
Figure 36 is a plan view which illustrates an absorbent product according to a working example of the present device.
Figure 37 is an enlarged sectional-view of the absorbent product shown in Figure 1.
Figure 38 is an enlarged sectional-view of elements of the inner sheet shown in Figure 1.
Figure 39 is a perspective drawing which illustrates an absorbent product according to another working example of the present device.
Figure 40 is a plan view which illustrates the absorbent product shown in Figure 39. and
Figure 41 is a sectional view of the essential part of the absorbent product shown in Figure 7.

### Best Mode for Carrying out the Invention

Hereinafter, a porous composite sheet of the present invention will be concretely explained by making reference to drawings.

In the present invention, as modes by which an A/B-component layer in which an A-component layer and a B-component layer coexist is formed, the following modes can be employed:
(1) Mode according to which an A-component layer and a B-component layer are laid on top of each other so as to form an A/B-component layer;

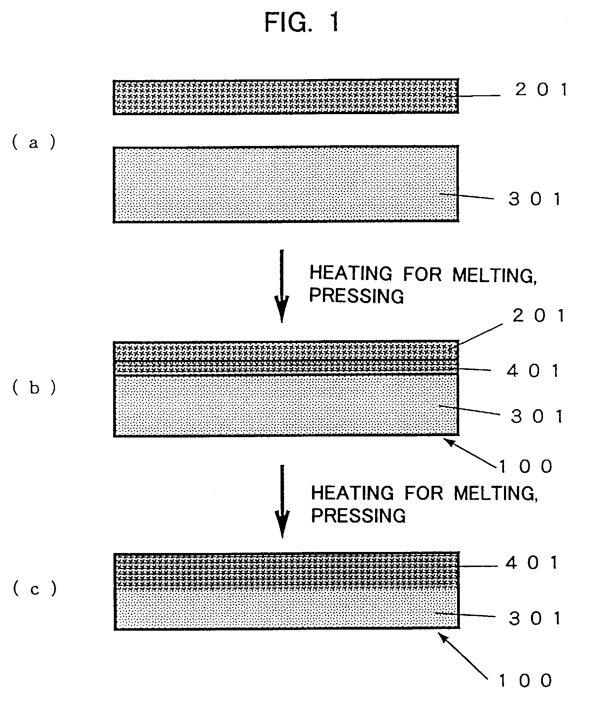

Figure 1 typically illustrates changes to a sintered state, in which a first porous layer, i.e. an A-component layer (201) and a second porous layer, i.e. a B-component layer (301) are heated under pressure in a state in which the first and second layers are laid on top of each other (see Figure 1(a)), so that A-component only is molten, whereby the A-component layer (201) is infiltrated into the B-component layer (301), so that a structure in which three layers of A-A/B-A coexist (see Figure 1(b)) is first formed. When the melting and infiltrating are further advanced, said structure results in a two-layers structure of A/B-B (see Figure 1(c)). By way of such processes, a molten layer of A-component and B-component (401) are produced, so that a monolithic porous sheet (100) is formed.

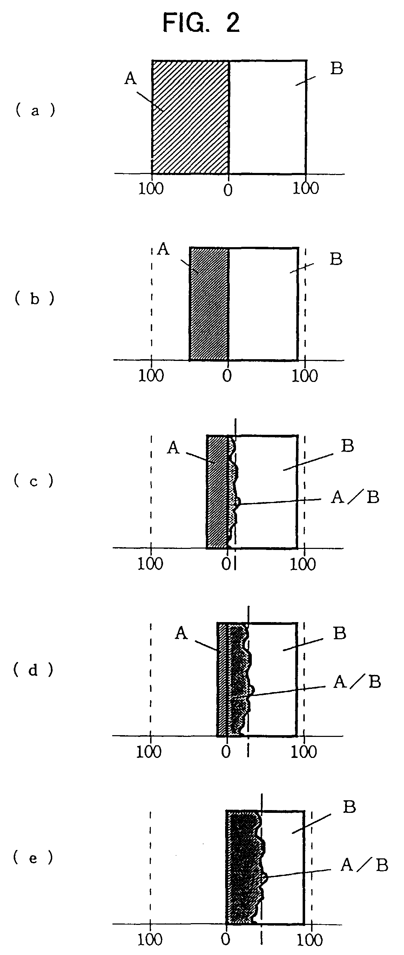

Figure 2 typically illustrates a progression process into a sintered structure. In the step (a) of Figure 2, an A-component layer and a B-component layer are merely laid on top of each other, and maintain the initial thickness (represented as 100) thereof, respectively. Then, when both are heated while pressed in the direction of the thickness, the decrease of the thickness of the A-component layer is remarkably decreased due to a thermal deformation of the constituents thereof (see the step (b)), although the decrease of the thickness of the B-component layer is a little. When a further heating is carried on in the state, A-component begins to infiltrate into the B-component layer (see the steps (b) to (d)) while the A-component layer is further pressed, and the A-component layer is finally substantially exhausted, and in the step (e) an A/B-component layer and the B-component layer only are left.
The present invention includes a layer structure such as is obtained according to each step from the step (c) to the step (e). An example of changes of the apparent specific gravity and thickness of each component layer at each step are shown in the following Table 2.

**Table 2**

| | | A | B | A+B |
|---|---|---|---|---|
| Step 1 A,B | Specific Gravity | 0.07 | 0.10 | |
| | Thickness | 60 µm | 100 µm | 160 µm |
| Step 2 A,B | Thickness | 35 µm | 95 µm | 130 µm |
| Step 3 A,A/B,B | Thickness | 30 µm | 90 µm | 120 µm |
| Step 4 A,A/B,B | Thickness | 10 µm | 90 µm | 100 µm |
| Step 5 A/B,B | Thickness | 0 µm | 90 µm | 90 µm |

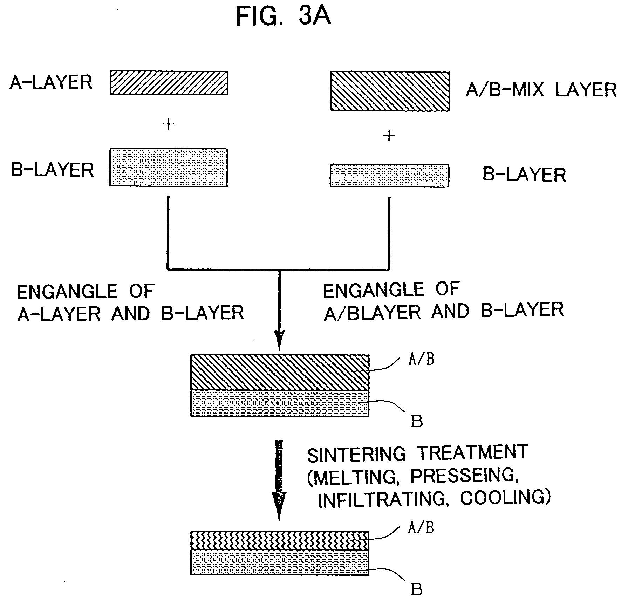

(2) Mode according to which a structure in which A-component and B-component are coexist is prepared, and the structure is sintered to a B-component layer;
In a sintered porous composite sheet of the present invention, it is necessary that part in which A/B-component coexist is integrated with a B-component layer, so that said composite sheet must have the same function as that of the above-mentioned structure in which the A-component layer is shifted into the B-component layer.
(2)-1 Mode according to which an A-component layer and part of a B-component layer are entangled so as to form a structure of A/B and B, and the part of A/B is sintered together;
   In this case, an A-component layer and a B-component layer are laid on top of each other at the step of a raw material sheet, and the A-component layer is entangled by a needle punch or a high-pressure water-stream so as to form a multiple-layer having a (A/B)-B layer-structure in which the form of an A-(A/B)-B layer-structure is also partially contained, and the multi-layer is heated and pressed under melting conditions of A-component so as to obtain a sintered composite of the present invention.
(2)-2 Mode according to which a mixed web of A-component and B-component is entangled with a B-component layer;
   When different fibrous webs are used, a mixed web of A-component and B-component is formed as an A/B-component layer (a first layer), and may be entangled with a B-component layer (a second layer) by a needle punch or a high-pressure water-stream so as to obtain a composite sheet having a two-layer structure of (A/B)-B.
   A process of forming a sintered structure as explained in the above items (2)-1 and (2)-2 is shown in Figure 3A. In this case, an effective binding can be provided by making A-component coexist in a B-component layer, wherein it is important to consider the mixing ratio (A/B ratio) of A-component to B-component so as to maintain the difference thereof between the first layer and the second layer. The A/B ratio of the first layer is 0.5 or more, preferably 1.5 to 5.0, while that of the second layer is 1.0 or less, preferably 0.5 to 0. When the A/B ratio of the first layer and that of the second layer come close to each other, for example, the design that heating is carried out merely at the side of the first layer while an apparatus having a cooling function is provided at the side of the second layer is desired.

When the A-component layer is rapidly molten, contractions by a volume change are rapidly caused, so that creases and/or non-homogeneous portions are liable to occur. Although it is also possible to provide a surface structure by intentionally utilizing the occurrence of such contractions, generally it is desirable that the surface is homogeneous. In order to prevent the occurrence of contractions as mentioned above, a treatment comprising the two steps of pre-thermal compression bonding and sintering is effective, while a mode according to which a structure of the A/B-component layer and the B-component layer is previously formed, and then sintering treatment is carried out is an effective one for stabilizing the shape.
(3) a mode according to which bicomponent fibers having A-component and B-component in one fiber are used as a material so as to form a first layer and a second layer, and both layers are sintered together so as to form an A/B-component layer; When a bicomponent fiber comprising a sheath which comprises an easily fusible component (A-component) and a core which comprises a slightly fusible component (B-component) is used, even if the sheath is molten, the structure of the core is maintained, so that heat contractions which occur with thermal treatment are also decreased, and productivity is also improved. A combination of a sheath/core is exemplified as follows:

| Core | Sheath |
|---|---|
| PET | PE |
| PP | PE |
| PET | Easily Melting PET derivatives |
| PP | EVA |

An example in which these bicomponent fibers are concretely utilized will be shown; for example, the first layer is formed from a web comprising a sheath/core fiber of PE/PET, while the second layer is formed from a fibrous web comprising 50% of PE/PET and 50% of rayon. The first layer and the second layer are superposed on top of each other, and sintered together at a temperature of the melting point of PE or more, and thereby a sintered composite sheet of the present invention can be obtained. Relations between the first layer and the second layer are that generally the first layer contains A-component in a relatively large amount, and the second layer contains B-component in a relatively large amount, so that a constitution in which the second layer has a relatively higher thermal stability is provided.

However, such relations between the first layer and the second layer are also obtained by using materials having the same constitution as each other, which will be explained, for example, by enumerating an example in which the above-mentioned bicomponent fiber is used as a material. Namely, a spunbond comprising a fine hydrophobic PE/PET-system bicomponent-fiber having a fineness of about 2 deniers is prepared as a first layer; a mixed web comprising a PE/PET-system bicomponent-fiber and a different fiber(s) is prepared as a second layer, wherein said bicomponent-fiber has a fineness of about 4 deniers, and is treated with a hydrophilicity-providing agent; and both layers are laid on top of each other, and heated and pressed from the side of the first layer, and thereby the PE component is shifted from the side of the first layer to the side of the second layer, so that a composite sheet which is sintered in a state close to adhesion can be formed.

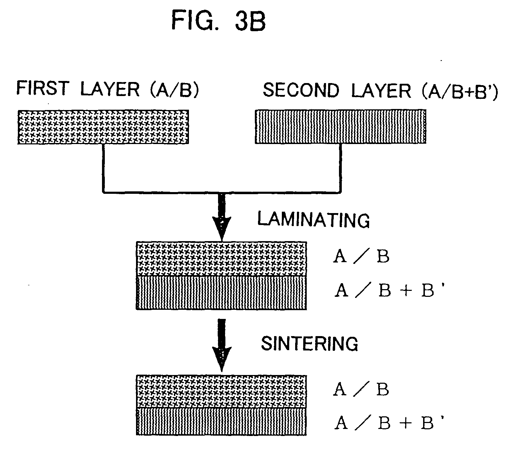

A process for forming a sintered structure as explained in the item (3) is shown in Figure 3B.

In a first typical example in which the characteristics of the sintered porous composite sheet are used, the characteristics that the composite sheet is water-resistant while infiltrates air and water-vapor, which is caused by a microporous structure thereof, are utilized. Thereby, a water-resistance up to about 500 mmH 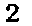 O which is practically warrantable is easily provided, while a high water-resistance at the same level as that of a homogeneous synthetic-resin film is not provided. Furthermore, air-permeability thereof is far excellent as compared with the level of what is called an air-permeable film, and is larger than 100sec/100cc.

In this specification, an estimation of water resistance and air-permeability is carried out according to the following methods:
Water Resistance (mmH 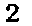 O): JIS L1092 (Low Water-Pressure Method)
Air-Permeability (sec/100cc): JIS 8117P (Gurley Method)

In order to sufficiently exhibit these merits, it is preferred to use a hydrophobic fiber having a fineness as small as possible, what is called a microfiber-web, as an A-component layer, while a material having high water-repellency should be used as a B-component layer.

Such a sintered sheet is an optimum raw-material for uses such as backsheet s of napkins, outdoor-sports wears, crease-resistant wears and diapers.

A porous composite sheet of the present invention has a wide range of application.

For example, a raw material having a microporous structure as mentioned above can be naturally utilized also as a biobarrier material, while for uses for which biobarrier properties are necessary, a high water-repellency of the surface rather than water-pressure resistance, as well as air-permeability are desired, in addition thereto, a high sweat-absorption power of the surface is desired.

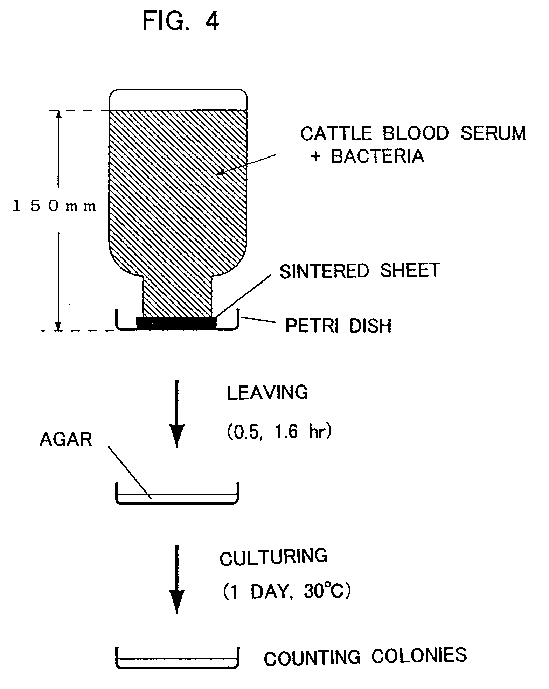

An estimation of biobarrier properties is represented by results which are obtained according to a method as shown in Figure 4. Namely, a test specimen was put on the opening of a glass bottle which contained a bacteria dispersion at a height of 150mm, a petri dish was put thereupon, the whole was inverted and was still stood for a period of 0.5, 1 and 6 hours, and thereafter, the petri dish was removed, an agar was injected thereinto, and the bacteria was cultured at a temperature of 30°C for a day, and colonies were counted.
- Used Bacteria:: Serratia Marcescens IFO 12468 (0.48 µm)
Pseudomonas Diminuta IFO 14213 (0.2 µm).
- Bacteria Dispersion:: 800ml of Blood Plasma of Cattle which was Diluted with Physiological Brine to three times
Bacteria Concentration: Approx.10 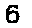 /ml.

In order to exhibit these biobarrier properties, it is desirable that a repellent microfibril layer is provided as an A-component layer, a hydrophilic cellulose fiber is provided as a B-component layer, and both layers are sintered together so as to form an A-(A/B)-B layer structure such as the A-component layer is a little left on the surface of the structure. Such a sintered porous composite sheet are fit for surgical or medical materials such as a surgical gown, a surgical drape and a mask, while are also fit for a filter material for a vacuum cleaner due to a porous multi-layer structure and an excellent crease effect.

In a second typical example in which the techniques of the sintered porous composite sheet are used, the composite sheet is utilized as the surface sheet of an absorbent product.

As important elements for estimating the function of the composite sheet as the absorbent product, absorbing speed, and one-way water-absorbing properties which do not turn back are enumerated. Conventionally, it has been said that a very thick structure is necessary for such a raw material having one-way water-absorbing properties. However, by using a sintered structure of the present invention, it comes to be possible to develop a water-absorptive sheet which has high-grade one-way water-absorbing properties and instantaneous water-absorbing properties such as absorbing speed is very fast.

As one example for realizing this purpose, it is desirable that a hydrophobic easily fusible web such as PP or PE in the form of a relatively thin layer is prepared as an A-component layer; a cellulose porous sheet which is excellent in hydrophilic property is selected as a B-component layer; said A-component layer is molten and infiltrated into said B-component layer, and thereby both layers are sintered together till a layer consisting of A-component only comes to be little left, that is, till the form of an (A/B)-B-component layer is obtained.

A thin sheet having a fine fineness and a specific weight of 20g/m 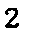 or less is desired as an A-component layer; for example, a spunbond comprising bicomponent fiber which comprises PP as a core and PE as a sheath. These sheets are drawn in a cross direction and in a machine direction, and thereby thin and soft ones are formed, so that said sheets are raw materials having a very high fitness. As a B-component layer, TCF, a cellulose nonwoven fabric such as Wenliese, tissue paper, an air-laid mat of wood-pulp, a synthetic fiber web which was treated to have hydrophilic property, and a sheet-like material having high water-absorbing property are suitable.

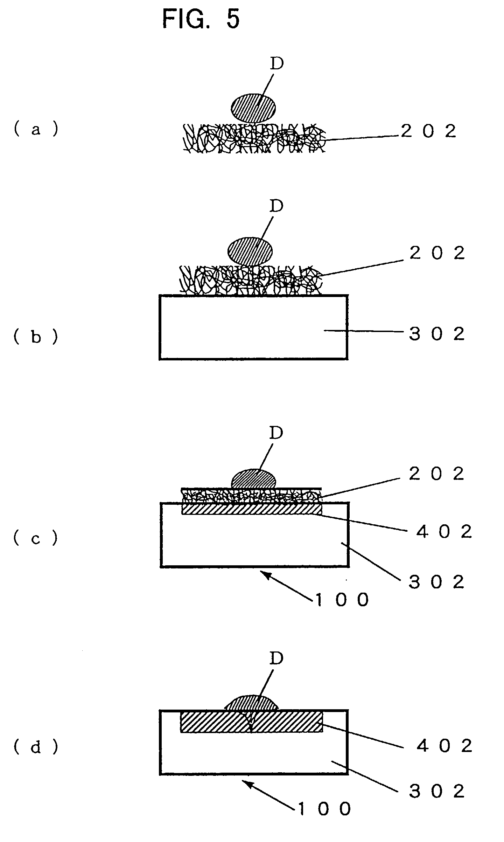

Figure 5 shows progress steps of sintering, and the change of state of the infiltration of a water-drop (D) which was put upon an A-component layer (202). Namely, Figure 5(a) shows the state that the water-drop (D) is put upon the A-component layer (202) which is an aggregate of fine fibers, wherein the water-drop (D) shows little permeability to the A-component layer (202). Figure 5(b) shows the state that a B-component layer (302) is laid on top of the A-component layer (202), wherein there are no changes of the water-drop (D). However, when part of A-component is sintered to B-component so as to form a sintered portion (402) as shown in Figure 5(c), a little permeability occurs on the surface of the A-component layer (202), while when a further sintering is highly advanced so as to almost completely shift A-component into the B-component layer (302), a remarkably high permeability is revealed.

Namely, a porous composite sheet which is sintered in a state as shown in Figure 5(d) presents a thin-film appearance on the surface layer thereof, and has a smooth surface without no fuzz. Furthermore, since the end of the hydrophilic component is exposed on the surface thereof out of pores which are innumerably present on the surface which has come like a hydrophobic film, the sheet very speedily absorbs water, so that water is transferred to the B-component layer (302) which is a lower layer. Therefore, when a water-drop is put thereupon, the water-drop is almost instantaneously absorbed, and moisture is little left on the surface. Furthermore, due to smoothness property of the surface, minute water-drop also is entirely absorbed without wiping up the same, a function just as a blotting paper is provided. Accordingly, by making the best use of these merits, the present sheet is applied to the following uses:
(1) blood-absorbing sheet material:
   - surgical tray, tray for removing drains for meat; and
   - absorptive-plug coating-material (for ophthalmology, or for cerebral surgery)
(2) sanitary material:
   - diffusion sheet or surface material of a physiological article;
   - surface cover material of a tampon; and
   - diffusion sheet or top sheet of a diaper.
(3) lint-free wipes.

The above has been explained about when a sintering-treatment is carried out all over the surface of sheets, while a sintered porous composite sheet having partially sintered area also has a broad use-range. In particular, a product in which a first hydrophobic porous layer and a second high water-absorbing porous layer are partially combined with each other has excellent merits as a member which is used as the area of an absorbent product such as makes contact with skin.

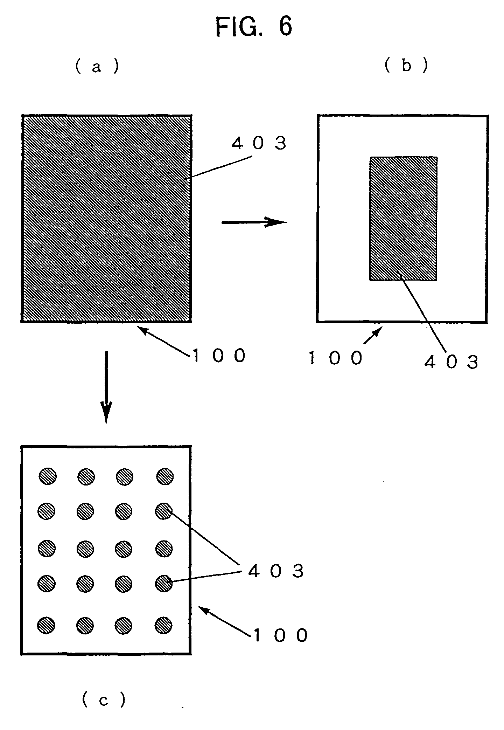

Figure 6 illustrates a distribution state of sintering treated area(s). A constitution such as a sintered porous composite sheet (100) in which a sintered area (403) is formed all over the surface as shown in Figure 6(a); a sintered porous composite sheet (100) in which a sintered area (403) is restrictedly and partially provided within the center area thereof as shown in Figure 6(b); or a sintered porous composite sheet (100) in which many sintered areas (403) are arranged in the morphology of spots as shown in Figure 6(c) can be provided.

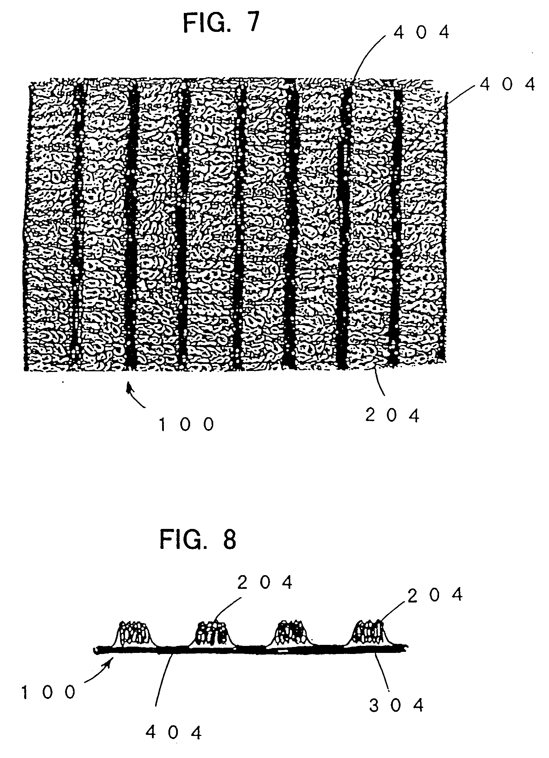

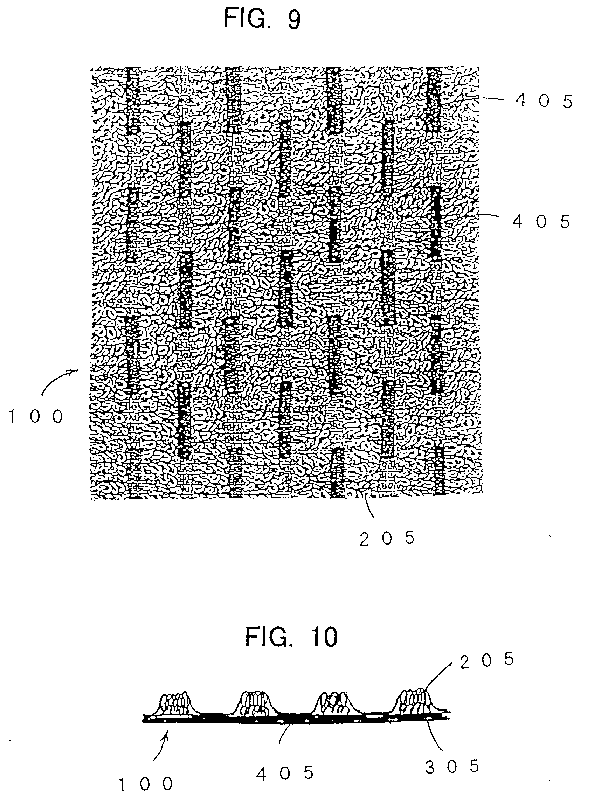

As further other embodiments, a sintered porous composite sheet (100) in which many sintered areas (404) are provided as shown Figures 7 and 8, wherein said sintered areas (404) are in the form of many continuous lines which are arranged on a parallel with each other so as to bind an A-component layer (204) and a B-component layer (304) which are laid on top of each other; or a porous composite sheet (100) in which many sintered areas (405) are provided as shown Figures 9 and 10, wherein said sintered areas (405) are in the form of many discontinuous lines which are arranged on a parallel with each other so as to bind an A-component layer (205) and a B-component layer (305) which are laid on top of each other, are enumerated.

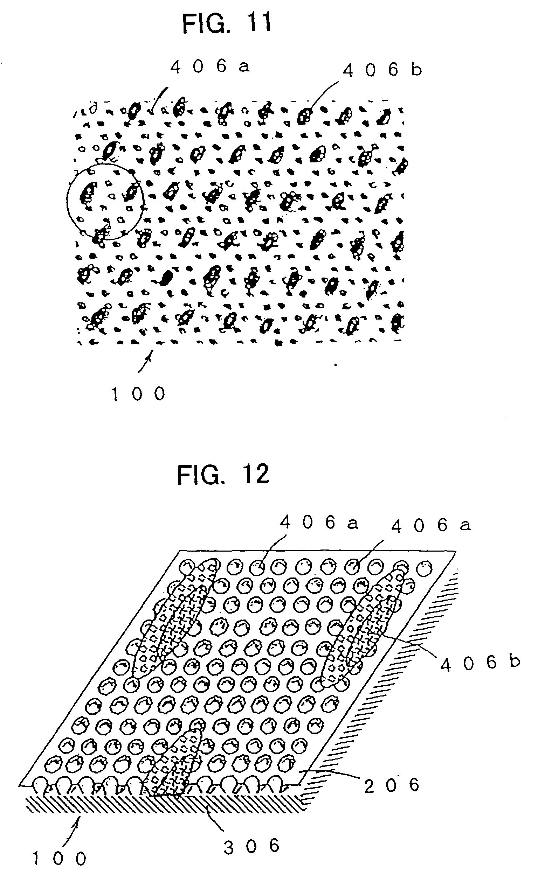

Besides, a structure in which many small sintered areas (406a) in the form of dots, and sintered areas (406b) each of which is larger than each of said small sintered areas (406a) and in the form of, for example, an ellipse dot, are arranged according to a proper distribution as shown Figures 11 and 12 can be employed.

With respect to an absorbent product, what is called a quilting process is employed, in which a top sheet and a transfer sheet are incorporated into one. In the quilting process, generally a binding in the form of points is employed, while a sintered area in the form of dots has to have an area. Even a small dot has to have an area having a diameter of 1mm or more.

A sintered porous composite sheet having a structure in which partial sintered areas are arranged according to a proper distribution as shown in Figures 7 to 12 speedily infiltrates and absorb moisture at the sintered areas, while hydrophobic property in the first porous layer is left as it is at non-sintered areas so that moisture can be shut out so as to always maintain a dry state.

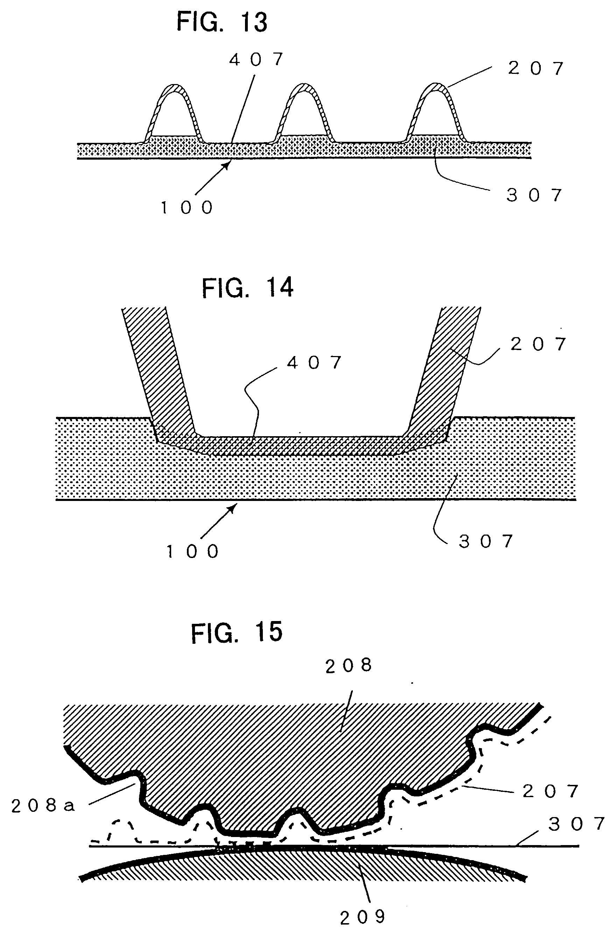

Effects as mentioned above come to be further remarkable by in three dimensions arranging the sintered areas and the non-sintered areas. Figures 13 and 14 illustrates a sintered porous composite sheet (100) having a structure in which a first wave-form porous layer (207) is positioned on a second flat porous layer (307), wherein both layers are sintered together at wave-trough areas of said first porous layer (207) so as to form sintered areas (407) in the morphology of lines or bands.

As one of uses for a sintered porous composite sheet as shown in Figures 13 and 14, a top sheet for an absorbent product will be exemplified. Due to a strong hydrophobic property of the first porous layer (207), no wave-crest areas thereof get wet with edgiest, and portions which keep in touch with skin are always kept dry, while the wave-trough areas thereof have a large water-permeability because the first hydrophobic porous layer (207) is sintered and united with the second hydrophilic porous layer (307) containing a cellulose material as shown in Figure 14. Therefore, a liquid which is gathered in the wave-trough is speedily infiltrated into the second porous layer (307) without a backward motion.

A sintered porous composite sheet having such a structure can be obtained according to various method. For example, as shown in Figure 15, the first porous layer (207) is stuck fast to a roll (411) on whose periphery surface a channel (411a) is provided by means of a suction or the like, so as to deform into a wave-shape; and a second flat porous layer (307) is laid on top of the first layer; and thereafter, both layers are sintered together at the wave-trough areas of the first porous layer, so that a sintered porous composite sheet (100) as shown in Figure 13 can be easily produced.

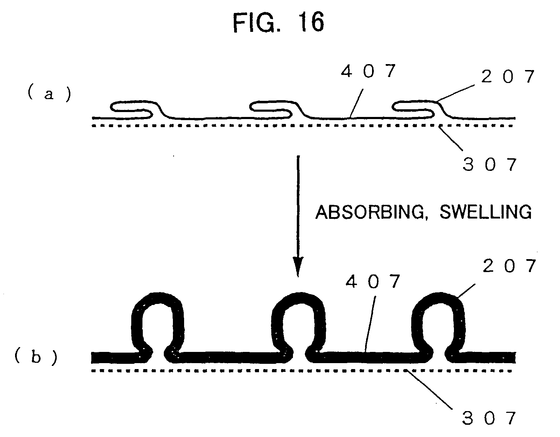

In a sheet-like composite absorbent having a structure as shown in Figure 13, a liquid provided on the first porous layer (207) is speedily diffused at sintered areas (407) which are formed in the wave-trough areas, and is absorbed and become to be fixed at, in particular, non-sintered areas, in the second porous layer (307). Furthermore, as typically shown in Figure 16(a), A-component (207) is crushed when no liquid is absorbed by the second porous layer (307). However, as a liquid is absorbed, the thickness of non-sintered areas of the first porous layer (207) is increased as shown in Figure 16(b), so that the wave-crest areas come to be protruded. Thereby, areas which keep in touch with skin are remarkably decreased in the whole absorbent, such an excellent use-feeling that the absorbent is always dry and the skin is separated from the liquid, is obtained.

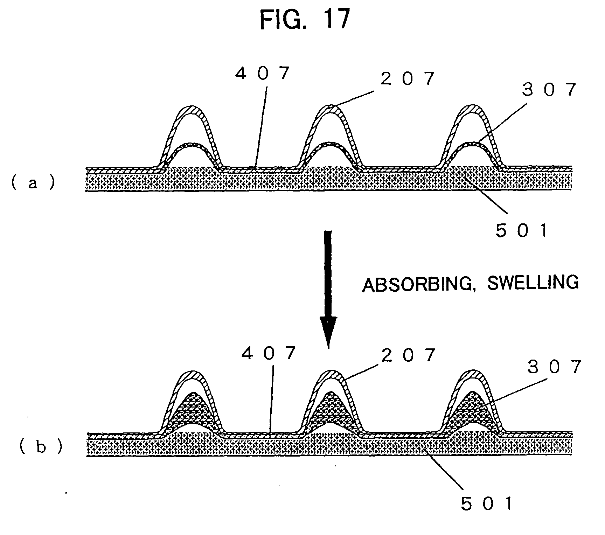

A sintered porous composite sheet as shown in Figure 13 can be used as a complete sheet-like composite absorbent body, when combined with a liquid-permeable sheet (501) laid on the back face of the second porous layer (307) as shown in Figure 17(a), wherein the second porous layer (307) acts as a top sheet, the second porous layer (307) as an absorbent body and the liquid-permeable sheet (501) as back sheet.

In the sheet-like composite sheet having a structure as shown in Figure 17, a better heave will occur, since the thickness of the porous second layer (307) increases with absorption of liquid as shown in Figure 16(b), in addition to that the heave of the wave-crest areas shown in Figure 16.

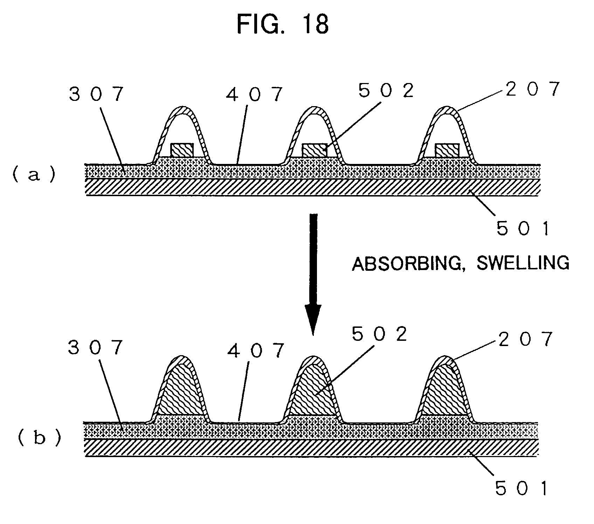

Furthermore, as shown in Figure 18(a), it is possible to accommodate a belt- or string-like polymeric absorbent (502) within each of the spaces formed by the first porous layer (207). This type of composite sheet is applicable to the appliances that requires a larger absorbing ability, since the absorbing ability of the polymeric absorbent (502) is added in addition to he absorbing ability of the second porous layer (307). The state that the polymeric absorbent (502) has absorbed and swelled is shown in Figure 18(b).

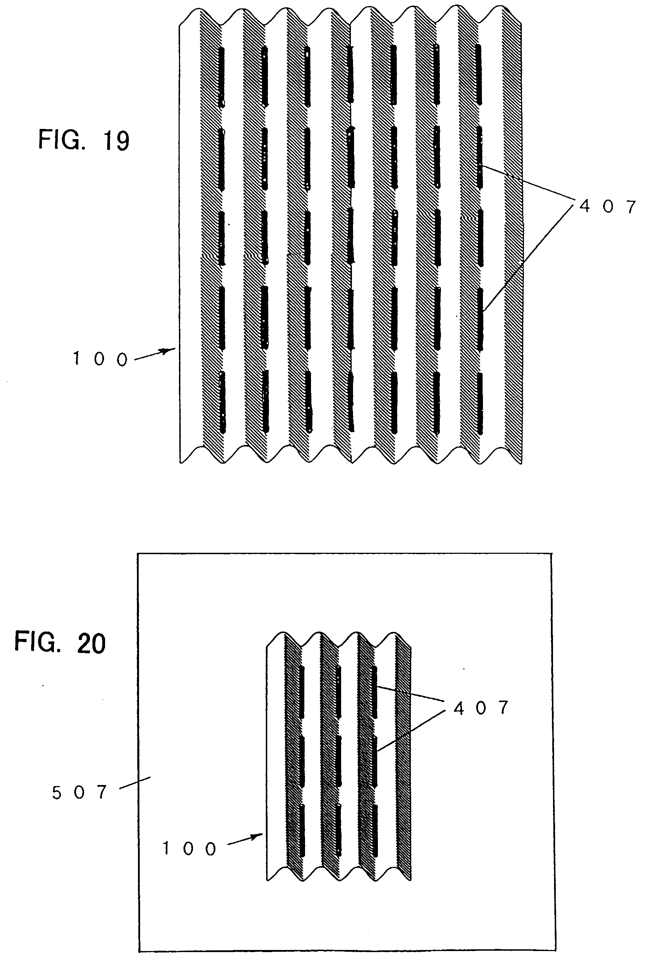

In the constructions shown in Figures 13, 17 and 18, sintered area (407) of the wave-trough areas may be in the form of a continuous line extending along the longitudinal direction of the wave-trough areas, or a plurality of short lines disposed in a line as shown in Figure 19. The sintered porous composite sheet (100) can have a smaller area than the absorbent material and joined together to cover part of the sheet-like absorbent (503) to form a sheet-like composite absorbent body.

The sintered areas (407) may be in the form of approximately round dots aligned in a line with suitable intervals.

If necessary, it is possible to form openings of proper size to allow a liquid to pass through the sintered areas physically.

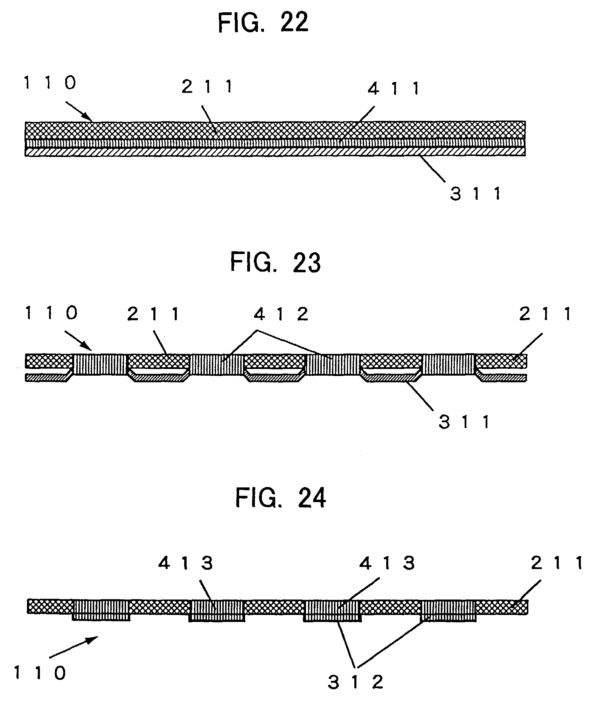

The porous composite sheet having a hydrophilic surface of to the present invention, as shown in Figure 22, first layer (211) comprising a hydrophobic material and a second porous layer (311) comprising a hydrophilic material are laid on and sintered together to form a water permeable composite sheet (110) having an A/B-component layer (411) therebetween.

Figure 23 shows a water permeable composite sheet (110) having areas where first hydrophobic porous layer (211) and second porous layer (311) exist independently, and areas where A/B-component layers (412) exist in a stripe. Further, Figure 24 shows a water permeable composite sheet (110) having areas where only first porous layer (211) exists and areas where A/B-component layers (413) exist, by arranging on the first porous hydrophilic layer (211) a plurality of second layers (312) having a suitable area in a desired distribution.

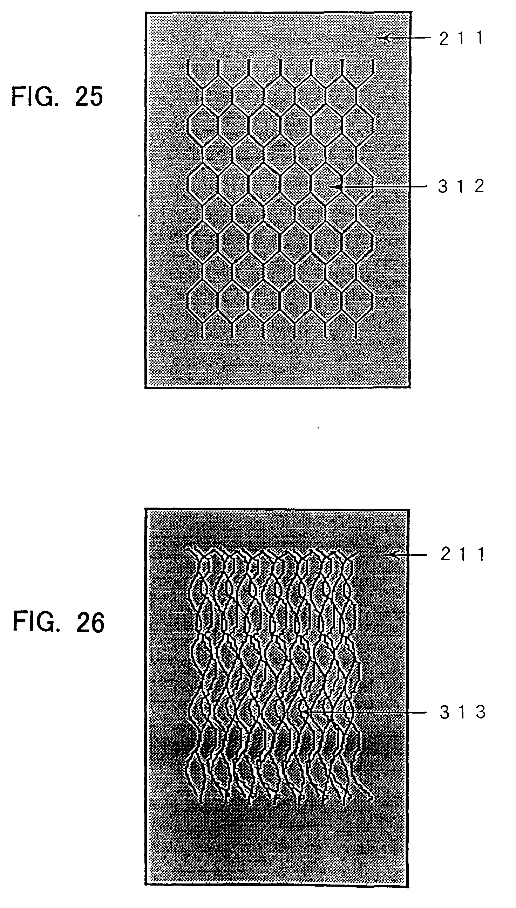

Figure 25 shows a water-permeable composite sheet (110) formed by laying on a first hydrophobic layer (211) such as PE/PET bicomponent spun-bond non-woven fabric a net which is produced by extruding a material such as PE to form a net and then subjected to a treatment for causing the net hydrophobic only on its surface, and then sintering together. Figure 26 shows a water-permeable composite sheet (110) using, in place of the PE net used in those shown in Figure 25, a PP foam (313) which is hydrophilicated and stretched in a width direction as a second layer. In case that the above B-component of net being hydrophilicated at its surface is used, water permeable regions are formed along the second layer.

When the first and second layers are sintered together, the higher temperature is applied, the larger water permeability is obtained at the formed sintered portion, while, the surface characteristics becomes near a film to be harder and the surface strength will increase. On the other hand, when the temperature and pressure are low, the texture is kept, but it tends to decrease the water permeability.

This means that the same materials will give water permeable composite sheets having different properties by adjusting the temperature and pressure applied. Thus, it is possible to obtain water permeable composite sheets having a suitable properties for the desired appliances by combining the above parameters.

In the sintering between the first layer and second layer, the sintered state of the sintered portion thus formed greatly depends on whether the necessary heat and pressure for sintering is applied to the first layer or the second layer.

Hereinafter, working examples of the present invention will be explained.

### Examples

### (Example 1)

### Combination of First Hydrophobic Layer and Second Layer:

### Preparation of A-Component Layer and B-Component Layer

A PE-made meltblown nonwoven fabric having a thickness of about 1mm (which has a specific weight of 15g/m², and an apparent specific gravity of 0.02g/m³) was prepared as a first easily fusible porous layer, namely, as an A-component layer. On the other hand, as a B-component layer, a spunlace nonwoven fabric having a thickness of about 1mm which was prepared by entangling 1.5d×35mm of polyester with a high pressure water-stream (which has a specific weight of 50g/m², and an apparent specific gravity of 0.05g/m³) was prepared.

### Conjugation and Sintering Treatment of A-Component Layer and B-Component Layer

A B-component layer was laid on top of an A-component layer so as to obtain a thickness of about 2.5mm. In this state, a silicone paper for separating was put on the A-component layer, and a heat treatment was carried out at a temperature of 160O°C under a pressure of 5kg/cm² for a period of about one minute by using a press equipment having a hot plate thereon. As a result, the surface smoothly came to be in a film state, so that a composite sheet which was pressed to about 0.8mm.

### Characteristics of Sintering Treated Articles

The water resistance of an A-component layer and a B-component layer which was determined in the state that the A-component layer and the B-component layer are laid on top of each other according to JIS 1092 (Low Water-Pressure Method) was about zero, while that of a sintering treated product exhibited a water resistance of 100mmH₂O, and an apparent specific gravity of 0.08g/0.8g/cm 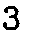 .

### (Example 2)

### Combination of First Hydrophobic Layer and Second Layer:

### Preparation of A-Component Layer and B-Component Layer

As an A-component layer, what is called a spotbond nonwoven fabric having a specific weight of 20g/m 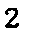 , which comprises PP-fibers (1.5d×35mm) and has an apparent specific gravity of about 0.06g/m 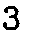 , was prepared. On the other hand, as a B-component layer, a sliced sheet of a polyester hard polyurethane foam having continuous foaming properties was prepared, which has an apparent specific gravity of about 0.03g/cm², a thickness of 3mm and a water-drop surface-tension of 75°C.

### Conjugation and Prehotpress of A-Component Layer and B-Component Layer

A heating and pressing apparatus comprising a pair of pair-rolls was prepared, wherein the upper roll is the one having a diameter of 300mm, and is provided with a heating apparatus with a solvent, the surface of said upper roll being chrome-plated and satin-finished. On the other hand, the lower roll was the one having a diameter of 400mm which was flat and chrome-plated, and was provided with no heating apparatus. A silicone separating-paper was put on an A-component layer and a B-component layer which were laid on top of each other, and said separating-paper, said A-component layer and said B-component layer were continuously passed at a surface temperature of 160O°C of the A-component layer at a rate of 3m/min. at a pressure degree of 5kg/cm², wherein the A-component layer was arranged at a heated side. As a result, a nonwoven fabric having B-component was sintered on the surface of urethane of A-component, so that a prehot-pressed sheet was obtained, whose surface was film-like. The water-pressure resistance said sheet was 20mmH₂O or less.

### Sintering Treatment

The above prehot-pressed sheet was again passed between the pair-rolls at a surface temperature of 200°C thereof at a rate of 1m/min. at a pressure degree of 15kg/cm², so that a sheet having a thickness of 0.5mm, whose surface was in the form of a smooth synthetic leather, was obtained. The water-pressure resistance of this sheet was increased to 300mmH₂O, and a contact angle of about 90O was shown.

### (Example 3)

### Sintered Sheet Combined with Cellulose Sheet:

### Preparation of A-Component Layer and B-Component Layer

As an A-component layer, a spunbond nonwoven fabric (having trade name "ELVES" made by UNICHIKA, and a specific weight of 25kg/m²) was prepared, said nonwoven fabric comprising filaments of a bicomponent fiber which comprises polyester as a core and polyethylene as a sheath. As a B-component layer, a sheet of airlaid-pulp nonwoven fabric (having trade name "QUINOCLOTH" made by HONSHU SEISHI, and a specific weight of 120g/m²) in which a polyacrylic polymer absorbent (trade mark "IM-3000" made by SANYOKASEI) is contained by 50g/m² was prepared.

### Conjugation and Sintering Treatment of A-Component Layer and B-Component Layer

An A-component layer was laid on top of a B-component layer, so that a thickness of about 3mm was obtained. A silicone separating-paper was put thereon, and heated with an iron to a temperature of about 180°C. The A-component layer, the B-component layer and the separating-paper were hot-molten from the side of the A-component layer while pressed by a body weight, so that a sheet in a state of a limp coat paper, which has a surface luster, was obtained. This sheet had a thickness of about 1mm.

### Water-Absorbing Property of Sintering-Treated Article

This sintering-treated sheet had a very high water-absorbing property, and thereby a liquid was little left after absorbing water.

| | |
|---|---|
| • water-absorbing speed | 2sec/100cc |
| • Rewet | 0.1g or less |

### Application to Tray for Raw Meat

Two cases of fillet steaks of beef (steak cut) which were packed in a polystyrene tray were prepared. The present steaks were somewhat blood-stained on the underside thereof. The above-mentioned sintered sheet was spread in one case, while a QUINOCLOTH was spread in another case, and then the cases were wrapped with a wrap, respectively, and were left as it is in a refrigerator for a period of 24 hours, and thereafter, both were compared with each other. As a result, in both cases, no blood was left, and regarding the sintered body, there was little difference between the upper surface and the contact surface of the sintered body, while some area whose color was changed into white was observed on the side which had been brought into contact with the QUINOCLOTH. Furthermore, regarding the QUINOCLOTH, the appearance that blood adhere between the meat and the sheet was observed, while regarding the sintered-body sheet, no such phenomenon was observed, and it was also demonstrated that the sintered-body sheet is excellent in separating property.

### (Example 4)

### Stretching Sintered Body:

### Preparation of A-Component Layer and B-Component Layer

According to meltblown method, an elastic nonwoven fabric having a specific weight of 40g/m² was prepared from a compound having 70 parts of SEBS (trade mark "CRAYTON RESIN") and 30 parts of EVA (trade mark "EVAFLEX") as main components, so as to provide an A-component layer. This web had an apparent specific gravity of 0.05g/cm³. As a B-component layer, what is called a spunlace nonwoven fabric (a specific weight of 30g/m²) was obtained, which was prepared by treating a parallel-card web with a high-pressure water, wherein said parallel -card web comprises 40 parts of a polyester fiber (1.2dx35mm), and 60 parts of a bicomponent fiber (trade mark "NSF-PT" made by DAIWA BOSEKI; 1.5dx35mm) which comprises a polymethylenepenthane having a strong repellent property as a sheath, and PP as a core. This nonwoven fabric had an apparent specific gravity of 0.08g/cm³. The nonwoven fabric of the B-component layer had an elongation property of about 20% (three times) in a cross direction (CD).

### Conjugation and Sintering-Treatment of A-Component Layer and B-Component Layer

A B-component layer was laid on top of an A-component layer, and both layers were prepressed under a pressure of about 5kg/cm² by using a mangle at an ordinary temperature. Even by a cold pressure, a temporary conjugation state was obtained.

The above conjugated body of the A-component layer and the B-component layer was led to a pair of heating rolls while the B-component layer was kept upwards, wherein the surface of the upper roll of the heating rolls had been chrome-plated, and had been heated at a surface temperature of 120O°C from the inside, while the lower roll had been made from a silicone rubber, and had been not heated, and had maintained a temperature of 70O°C with a remaining heat from the upper roll. The conjugated body between the pair of rolls was heated and pressed under a pressure of 10kg/cm² , while heated from the side of the B-component layer, wherein the pass speed was 3m/min.

This treatment made the A-component layer in the form of an almost complet e film so as to form a pliant sheet having an opal-like color. This sintered porous composite sheet had the following physical properties. Furthermore, this composite sheet showed excellent stretching properties; a hysteresis curve after drawing by 15 0% had an elastic recovery factor of 85%.

| | |
|---|---|
| * Apparent Specific Gravity | 0.12g/cm³ |
| * Air-Permeability (JIS P8117 Gurley Method) | 30sec/100cc |
| * Water Resistance (JIS L1092 Low Water-Pressure Method) | 400mmH₂O |

### Application of Sintered Articles to Diaper

A test such as the above sintered porous composite sheet is applied to a back sheet of "slipping type" underpants for babies.

As a blank, a commercial pants-type diaper in which PE-made air-permeable film was used, wherein the potion of the back sheet of the blank was replaced with a sintered sheet so as to form a sample, and five pieces of such a sample to every one of ten babies were worn so as to carry out a comparative test. As a result, regarding a leak from the back sheets, no significant differences as compared with the blanks were observed, and the wearing properties and the soft touch thereof could be particularly appreciated.

### Application as Ground Fabric of Pap Material

The side of the B-component layer was coated with a paste having a feeling of coolness which has arnicatincture, glycol salicylate and Q-menthol as main components so as to form a pap material, and the function thereof was tested. This pap material was thin and pliant, and caused no leak of chemicals, and was excellently appreciated.

### Application to Operation Gloves or Cap in Which Biobarrier Properties Are Utilized

Since a sintered porous composite sheet according to the present Example has a high biobarrier property, said composite sheet was used as an operation glove or cap which need high water resistance, air-permeability and elasticity, and thereby it was observed that the composite sheet has a remarkably improved wear-comfortability as compared with that of a conventional rubber glove. The estimation results of biobarrier properties which were obtained according to Bacteria-permeability test method as shown in Figure 4 will be shown in the following Table 3.

**Table 3**

| | Number of Raw Fungus | | | | | |
|---|---|---|---|---|---|---|
| Srain | P.diminuta | | | S.marcescens | | |
| Time(hr) | 0.5 | 1.0 | 6 | 0.5 | 0.1 | 6 |
| Example 4 (Sintered Composite Sheet) | <10² | <10² | <10² | 0 | 0 | <10² |
| B-Component Layer (Spunlace Nonwoven Fabric) | 10⁵ | 10⁶ | 10⁸ | 10⁴ | 10⁶ | 10⁸ |

### (Example 5)

### Sintering Treatment by Forming A/B-Component Layer:

### Preparation of A-Component Layer and B-Component Layer

A bicomponent staple fiber (1.5dx35mm) which comprises PP as a core, and PE as a sheath was used so as to prepare a card web of 20g/m² as an A-component layer, while a card web having a specific weight of 30g/m² as a B-component layer was prepared from staple fibers comprising polyester (3dx54mm).

### Formation of (A/B)-B-Component Layer

A web of A-component layer was laid on top of a web of B-component layer, and an A/B-component layer was entangled by a high pressure water-stream, and thereafter, was subjected to a hot air drying so as to prepare a multi-layer having the constitution of an (A/B)-B-layer.

### Sintering Treatment

The above multi-layer web was subjected to a sintering treatment at a temperature of about 180°C by using an iron in the same way as Example 3, so that a pliant sintered porous composite sheet was obtained.

### Tape Landing Zone Utilizing Sintered-Film Surface

A commercial adhesive tape for a diaper was used, and the adhesive tape and the above sintered porous composite sheet were united so as to test re-release properties. As a result, the composite sheet could stand the re-application test of the adhesive tape over tens times.

Recently, a nonwoven fabric has been used as, for example, a back sheet of a diaper for babies, while by the selection of a nonwoven fabric to be used and the sintering-treatment of the components of an absorbent, and by thermo-compression bonding treatment, such a tape landing zone can be formed without applying a new special film thereto.

### (Example 6)

### Sintering-Treatment with A/B-Component Layer Formation:

### Preparation of Mixed Web of A/B-Component

60 parts of an easily melting bicomponent fiber (trade name "MELTY" made by UNITIKA, LTD.) as an A-component material and 40 parts of viscose rayon (1.5dx45mm) as a B-component material were mixed and blended and subjected to a carding so as to prepare a mixed web having a specific weight of 30g/m², wherein said bicomponent fiber comprised polyester as a core and a polyester derivative as a sheath. The A/B ratio was 1.5.

On the other hand, as a B-component layer, a card web (a specific weight of 20g/m²) comprising a viscose rayon (1.5dx45mm) only was prepared.

### Preparation of (A/B)-B-Multi-Layer Body

The A/B-mixed web was laid on top of the rayon web of the B-component layer, and subjected to a high-pressure water-stream treatment and then a hot-air drying, so as to prepare a combined multi-layer body having an (A/B)-B-layer formation.

### Sintering Treatment

The above multi-layer body was subjected to a sintering-treatment at a temperature of 180°C by using an iron in the same way as the one in Example 3, so that a pliant sheet whose surface came to be a film was obtained. When water was dropped on the surface of the A-component layer of this sheet, water was instantaneously absorbed.

### (Example 7)

### Application to Raw Material for Surgical Gown Having Biobarrier Property:

### Preparation of Raw Materials for A-Component Layer and B-Component Layer

A silicone resin was added to a combination of PP/PE so as to prepare a tow of a fiber having water repellency and cleavage property (trade mark "DF-72" made by DAIWABO CO.,LTD.), and said tow was cut into a size of 2dx5mm in such a wet state as said tow was wetted with water containing a surface active agent, so as to prepare a chip as a raw material of an A-component layer.

On the other hand, as a B-component layer, a spunlaced nonwoven fabric (30g/m²) was prepared, wherein an antibacterial polynosic rayon (trade mark "KITOPOLY" made by FUJIBO CO.,LTD.) having a size of 1.5dx35mm was used as a raw material, and subjected to a water stream entangling so as to obtain said nonwoven fabric.

### Preparation of Layered Product of A-Component Layer and B-Component Layer

The above fiber having cleavage properties was made to be slurry, and was passed through a wet defibrillator for pulp so as to prepare a slurry of an extra fine fiber. On the other hand, a cylinder paper machine was used to provide the above spunlaced nonwoven fabric along a cylinder mold, and the slurry was provided thereon so as to form a mat of extra fine fibers. The mat was about 30g/m² in weight.

Then, the mat was treated by using a series of nozzles having hydraulic pressure of the three steps of 40kg/cm², 60kg/cm² and 80kg/cm² in a wet state; the surface of the spunlaced nonwoven fabric and the extra fine fiber were entangled; and then was dried with a Yankee drum dryer, so that a layered product of the A-component layer and the B-component layer was obtained, which had a surface gloss and was in a film state, while the morphology of a fiber was also observed therein.

### Sintering-Treatment

The above sheet was passed through a hot calendar-roller having a satin finished surface, and subjected to a sintering treatment at a temperature of 160O°C under a pressure of 15kg/cm², so that a soft sheet whose surface was transformed into an artificial leather state was obtained.

### Estimation of Characteristics of Sintered Workpiece

According to a method as mentioned above, the following properties were examined:

| | |
|---|---|
| • Air-Permeability | 15 sec/100cc; |
| • Water-Resistance | 100 mmH2 O; |
| • Contact Angle | 105 degrees (Water repellency was observed); and |
| • Bacteria-Permeability Test | (Kind of bacteria was P.diminuta only). |

Results are shown as the following Table 4.

**Table 4**

| | Number of Bacteria (Pieces) | | |
|---|---|---|---|
| Time(hours) | 0.5 | 0.1 | 6 |
| Sheet Before Sintering (Multi-Layer From Yankee) | Approx. 10³ | Approx. 10³ | Approx. 10⁴ |
| Sintered Sheet | 0 | 0 | 0 |

### Test for Application to Surgical Gowns

Surgical gowns were sewn with the above porous composite sheet, and a wearing test was carried out, according to which each of five surgeons put on the surgical gown so as to be compared with a conventional common nonwoven fabric gown (made by SONTALA, LTD.). According to their estimation for said composite sheet gown, it was demonstrated that a wearing comfort is the same as that of the SONTALA gown while a close and/or sultry feeling is little felt.

Furthermore, blood was jetted under pressure so as to hit against the surface of the gown, and the staining of blood under pressure on the gown was checked, so that since the sintered porous composite sheet gown shed blood, the staining thereof was not observed, while stains of blood were observed on the SONTALA product.

### (Example 8)

### Application to Diffusion Sheet or Absorbent Sheet:

### Preparation of A-Component Layer

A spunbond nonwoven fabric (trade mark "ELVES" made by UNITIKA, LTD.; 30g/m²) was drawn by 1.2 times in a cross direction and by 1.5 times in a machine direction in a hot air processor which had been heated to about 130°C so as to prepare a spunbond nonwoven fabric as an A-component layer, wherein said first nonwoven fabric comprised an easily fusible bicomponent fiber filament comprising polyester as a core and polyethylene as a sheath; and the final nonwoven fabric was drawn and deformed into a light weight fabric of about 16g/m².

### Formation of Composite of A-Component Layer and B-Component Layer

A paper machine of a short-mesh type was used, and a spunbond as an A-component layer was fed along a stainless net, and a B-component layer of 100g/m² was formed thereon, wherein said B-component layer comprised a soft wood pulp having a relatively long fiber. After removing water from both layers, both layers were further subjected to a jet of a high-pressure water of 70kg/cm² in a wet state on the net so as to entangle the spunbond and the pulp layer and to remove water therefrom and dry the same, so that a multi-layer body sheet (I) comprising an (A/B)-B-component layer was formed.

### Sintering-Treatment of Multi-Layer Body(I)

The above multi-layer body ( 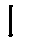 ) was passed through hot-calendar rollers having a surface temperature of 180°C under a pressure of 20kg/cm², and thereby a sintered sheet whose surface was deformed into a film and had a coat-paper-like gloss was obtained. This sheet was remarkably excellent in water-permeability, and the surface thereof had a preferable wiping property; for example, when 5cc of water was dropped on a flat glass and was wiped with the sintered sheet, even fine water-drops could be thoroughly absorbed.

### Application to Diffusion Sheet of Sintered Sheet Napkin

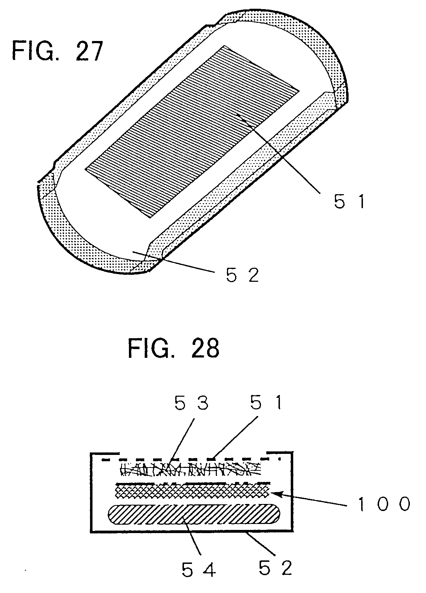

The above sintered porous composite sheet was incorporated into a sanitary napkin, as a diffusion sheet so as to prepare a sample as shown in Figures 27 and 28. In Figures 27 and 28, the reference numeral 51 shows a top sheet, 52 shows a back sheet, 53 shows a cushion web which is arranged downward the top sheet (52), and 54 shows an absorbent, wherein the sintered porous composite sheet (100) was arranged between the cushion web (53) and the absorbent (54).

The absorbing property of the sanitary napkin was tested. As a result, the absorbing speed and diffusion property were increased and an improved area-utilization factor was observed.

### Application of Sintered Sheet as Tip of Columnar Absorbing Bar

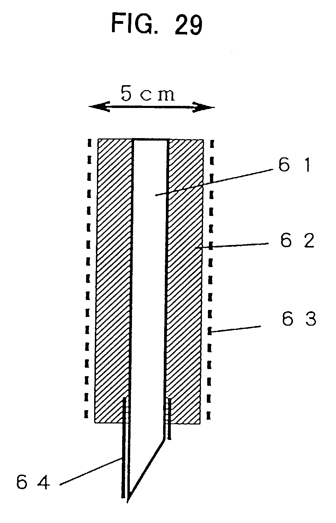

A surgical body-fluid absorbing bar having a structure as shown in Figure 29 was prepared. This absorbing bar comprises a bar body (61) whose tip was obliquely cut, said bar being made of TCF (a cellulose spunbond made by NIMUTA CHEMICAL, LTD.); a tissue paper (62); a perforated polyethylene film (63); and the sintered porous composite sheet (64), wherein the periphery of the bar body (61) was coated with the tissue paper (62), the paper (62) was coated with the polyethylene film (63), the tip was projected from the coating and was wound with the above sintered porous composite sheet (64).

It was observed that the surgical body-fluid absorbing bar had excellent improved effects on points such as absorbing speed, shape-holding property of the tip, the prevention of the occurrence of lint.

### Preparation of Multi-Layer Body (II) Obtained by CMC-Treatment of Multi-Layer Body (I)

The above multi-layer body (I) was immersed in a mixed liquid which comprises 10% of sodium hydroxide, 35% of potassium monochloroacetate, 1% of epichlorohydrin, and 54% of water for a period of one minute, and thereafter the multi-layer body (I) was maintained at a temperature of 60°C for a period of four hours so as to be partially transformed into a CMC. Then, the multi-layer body (I) was immersed in an aqueous methanol solution of 70%, and was dried with methanol of 100%. The degree of substitution of carboxymethyl group was 0.47. The pure-water absorbing amount of this CMC-treated multi-layer body (II) was 27g/g.

### Sintering-Treatment of Multi-Layer Body (II)

The above CMC-treated multi-layer body (II) was treated with a hot-calendar rollers having a surface temperature of 180°C, under a pressure of 15kg/cm², and was subjected to a sintering-treatment. The surface was deformed into a film, and had a coat-paper-like morphology.

### (Example 9)

### Partially Sintered Formation in Which Top Sheet, Absorber and Back Sheet Are United:

### Preparation of Stretching Back Sheet

A cellulose nonwoven fabric and an elastic film were combined with each other so as to prepare a composite stretching back sheet, wherein said cellulose nonwoven fabric is a spunlaced nonwoven fabric which was prepared by introducing a parallel card web (a specific weight of 40g/m²) which was obtained from a viscose rayon staple (1.5dx35mm), into a high-pressure stream entangling apparatus, and subjecting to a stream entangling treatment and drying. This nonwoven fabric has an elongation property of about 200%in a cross direction (CD).

As an elastic film, an elastic film comprising T.P.U. and having a specific weight of 40g/m² was prepared. the film and the above nonwoven fabric were laminated to each other all over the surfaces thereof with a hot-melt so as to obtain a back sheet which has a stretching property within 150%, wherein the laminated cellulose nonwoven fabric functions as a B-component layer in the present working example.

### Preparation of A-Component Layer

A spunbond (trade mark "ELVES" made by UNITIKA, LTD.; 26g/m²) comprising a bicomponent filament fiber comprising polyester as a core and polyethylene as a sheath was prepared.

### Partially Sintering Treatment of A-Component Layer and B-Component Layer

A wave crest was formed in the above spunbond (the A-component layer) along the above-mentioned channel (11a) as shown in Figure 15, while the upper side of the nonwoven fabric was filled with SAP (trade mark "IM-3000" made by SANYO CHEMICAL INDUSTRIES, LTD.) and a granulated composite comprising peat-moss and acetate; the cellulose section (the B-component layer) of the back sheet was laminated thereto; and contact areas were heated at a temperature of 180°C under a pressure of 15kg/cm² at the side of the urethane film having heat resistance, so that the A-component layer was molten and bonded under a pressure so as to form band-like sintered areas. Thereafter, the whole was compressed in a cooling state so as to be deformed into a thin sheet shape as shown in Figure 16(a). The composite of SAP/acetate/peat-moss was deformed into a mat-shape under compression; and was conjugated to the surface of the B-component layer due to the compatibility with cellulose and due to the effusion of the hot-melt which was used for conjugating the back sheet. Typically, a formation as shown in Figure 18(a) was provided.

As mentioned above, an absorbent formation in which all of the required functions are consolidated is obtained, wherein the A-component layer functions as a top sheet, and the B-component layer functions as a diffusion sheet, an absorbent and a back sheet. When such a formation is applied to a diaper for babies, an excreted urine is speedily infiltrated into a B-component layer through sintered areas and is diffused, so that the urine is absorbed and fixed in an absorbent having a polymer absorbent as a main component. As a result, the formation is deformed into a morphology as if the wave crest was stood erectly. Actually, the crest is changed from such a state as brought down under compression as shown in Figure 16(a) to such a state as risen up due to a swelling as shown in Figure 16(b).

Since the wave crest portions are hydrophobic, there is no liquid leakage from the crest portion, and the skin of a baby keeps in touch with this portion and the skin is isolated from the absorbing surface, so that the skin is never stained with an eliminated urine or feces, and thereby an ideal state can be maintained.

### (Example 10)

### Preparation of A-Component Layer and B-Component Layer

A card web was prepared as an A-component layer, wherein the card web comprises a high contractile bicomponent fiber (PNE fiber made by DAIWABO CO.,LTD.) of a PE/PP side-by-side type having 2dx45mm, while a viscose rayon 30g/m² having 1.5dx45mm was prepared as a
B-component layer.

### Formation of Three-Layers Structure by Water-Stream Entangling of A-Component Layer and B-Component Layer

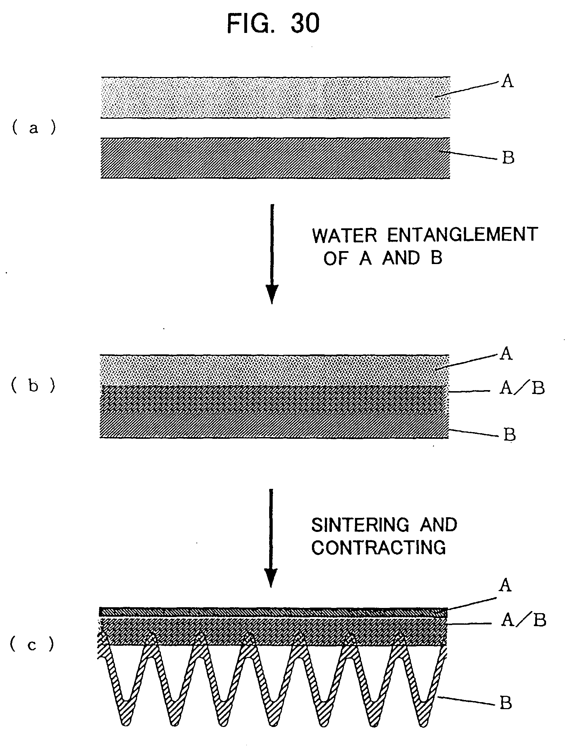

The above A-component layer and B-component layer were laid on top of each other, and were passed through a nozzle line comprising three high-pressure stream nozzles (30kg/cm², 70kg/cm², 75kg/cm²) on a porous cylinder which was provided with a dehydrator so as to subject to an entangling treatment, and then was subjected to a hot air drying at a temperature of 80°C, so that a multi-layer nonwoven fabric having about 45g/m² was formed (Figure 30(a)). This nonwoven fabric had a structure which is typically illustrated as shown in Figure 30(c).

### Heat Contraction and Sintering-Treatment

The above multi-layer nonwoven fabric was subjected to a contracting treatment in hot air at a temperature of 130°C under no stretch as a first step treatment, so that a three-dimensional nonwoven fabric having a specific weight of 100g/m² was obtained, wherein the A-component layer kept a relatively flat state while the B-component layer had a loop-shaped fold structure.

Furthermore, as a second step, a combination of a flat roller having a temperature of 180°C to which a Teflon (trade name) coating had been applied and a silicone rubber roller was prepared, and was passed through the rollers under a pressure of 4kg/cm² so that the flat surface of said three-dimensional nonwoven fabric could make contact with the flat roller, and the side of a folded surface could make contact with the silicone rubber roller. Thereby, A-component and B-component were sintered, a sintered porous composite sheet having a structure as typically shown in Figure 30(b).

### Utilization for Female Member of Velcro Zipper

The surface of the B-component layer having a three-dimensional loop structure was combined with a formed film whose surface has many hook-shaped prickles (made by 3M CO.,LTD.) so as to form a binding means for a diaper, wherein said surface of the B-component layer was used as a female member of a Velcro zipper while said formed film was used as a male member thereof. This zipper had remarkably high degree of coupling and was an excellent one.

Incidentally, when said composite sheet is used as a binding area of a non-returnable diaper in which said composite sheet is used as the female member, it is necessary to make the female member patch-shaped and to bind the same to a back sheet or the like by using an adhesive compound. A flat film is formed on the surface by sintering, and thereby the film-formed surface comes to be stably coated with an adhesive compound or the like, and furthermore a uniform conjugation with the adhered surface comes to be realized. Such a surface-sintering-treatment can be combined with a pretreatment in the process of an adhesive-compound coating.

### Application to Storage Carrier of Powdered Polymer Having High Absorbing Property

A multi-layer sheet having the folded surface in the present working example has both voids due to a large fold structure thereof and an instantaneous absorbent of a sintered surface. When such two characteristics are utilized, a powdered polymer having a high absorbing property can be relatively stably stored in an large amount to 20g/100cm² in the fold loop, and thereby, a very compact absorbent which is excellent in absorbing property and diffusive property can be industrially produced.

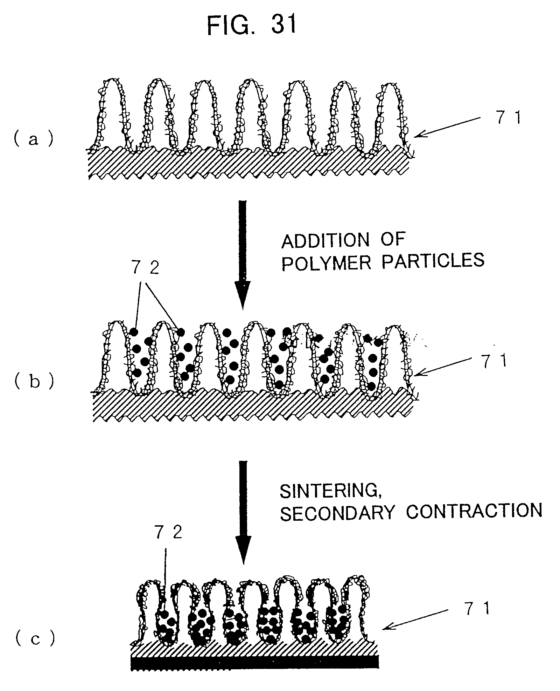

Namely, as shown in Figure 31(b), the powdered polymer (72) having a high absorbing property is introduced into the hollow of the multi-layer sheet (71) so as to be stored, wherein as shown in Figure 31(a), the multi-layer sheet (71) has a fold loop-shaped surface which was caused through the first contraction treatment. Since the powdered polymer (72) is easily florid off in a state as it is, a further contraction is advanced, while the surface is subjected to a sintering treatment, so that the space between the adjacent crests becomes narrow, and thereby, the movement of the polymer particle (72) is controlled and comes to be stably maintained in the storage sites. Furthermore, if necessary, the powdered polymer (72) is more stably fixed by absorbing moisture in some amount. Alternatively, the surface of the fold sheet is coated with a tissue, a film or the like, so that the effect of preventing the falling of the powdered polymer (72) is further improved.

### (Example 11)

### Sintered Porous Composite Sheet Combined with Cellulose Absorbing Sheet:

### Preparation of A-Component Layer and B-Component Layer

A spunbond nonwoven fabric (trade name 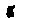 ELVES" made by UNITIKA, LTD.; 25g/m²) comprising a bicomponent fiber filament was prepared as an A-component layer, wherein said fiber filament comprises polyester as a core and polyethylene as a sheath. On the other hand, a sheet of trade name 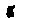 TEXEL" (a specific weight of 100g/m²) made by Shin-Oji Paper CO.,LTD. was prepared as a B-component layer, wherein said sheet is a sheet-shaped absorbing material which was formd by stream-entangling a wood-pulp and a PP-spunbond and was excellent in dimensional stability, and whose physical properties were enforced.

### Conjugation and Sintering-Treatment of A-Component Layer and B-Component Layer

The A-component layer was laid on top of the B-component layer, so that a thickness of about 4.5mm was obtained. A silicone released paper was put on the A-component layer, and was heated with an iron at a temperature of about 180°C under a body-weight from the side of the A-component layer for a period of about one minute. Thereby, A-component and B-component were sintered, so that a pliant coat-paper-like sheet having a surface gloss was obtained. This sheet had a thickness of about 2.1mm.

### Water Holding Property and Low-Dust Property of Sintered Porous Composite Sheet

This sintered porous composite sheet had a very speedy absorbing property, and after water was absorbed thereby, little water was left thereon. Furthermore, dust from the absorbing layer was little observed. Determined results for these performances are as follows:

| | |
|---|---|
| Water-Absorbing Speed | 2sec/100cc |
| Rewetting | 0.01g or less |
| Dust from Workpiece | 0.01g or less |

### Application of Chemical to Sintered Porous Composite Sheet

As a dust-absorbing material of wipes for a clean room, moisture-holding agents such as an aqueous polyethylene glycol solution and an aqueous organic carboxylic acid solution were absorbed in the B-component layer in an amount of 10g/m².

### Application to Wipes for Clean Room

A dust-absorbing test was carried out in a clean room. As a result, after many times repeated uses, no damage and no pucker were caused, dust and coarse particulate were stably adsorbed thereon, and no falling from the main body was observed. Furthermore, moisture on coarse particulate was also removed, and it was demonstrated that the composite sheet is also excellent wiping property.

### (Example 12)

### Top Sheet in Which Bicomponent Fiber Incorporated:

### Preparation of Hydrophobic A-Component Layer

A spunbond nonwoven fiber (trade name "ELVES" made by UNITIKA, LTD.) comprising a bicomponent fiber which comprises PET as a core and PE as a sheath was prepared as an A-component layer. This nonwoven fiber had a formation denier of about 2 deniers, a specific weight of 20g/m², an apparent specific gravity of 0.09g/m³ and water-repellency, while no water-permeability was observed under normal pressure.

### Preparation of Hydrophilic B-Component Layer

A card web of 25g/m² comprising a bicomponent fiber staple(trade name "SOFIT" made by KURARAY CO.,LTD.) of 3dx51mm, which comprises PET as a core and PE as a sheath and was made to be hydrophilic, was used, so as to prepare a thermal-bond nonwoven fabric according to Spotbond method. This nonwoven fabric was a soft-bulky one having an apparent specific gravity of 0.06g/m³, and was remarkably excellent in water-absorbing property.

### Sintering-Treatment of A-Component Layer and B-Component Layer

The above A-component layer and B-component layer were laid on top of each other, and according to a pattern as shown in Figure 26, only an area to be formed into an A/B-component layer was heated and pressed at a temperature of 130°C under a pressure of 10kg/cm² from the side of the B-component layer, so as to use as the surface material of a non-returnable diaper. As a result, the heated and pressed area was almost completely molten and conjugated to such a degree such a surface destruction was caused.

### Application to Diaper

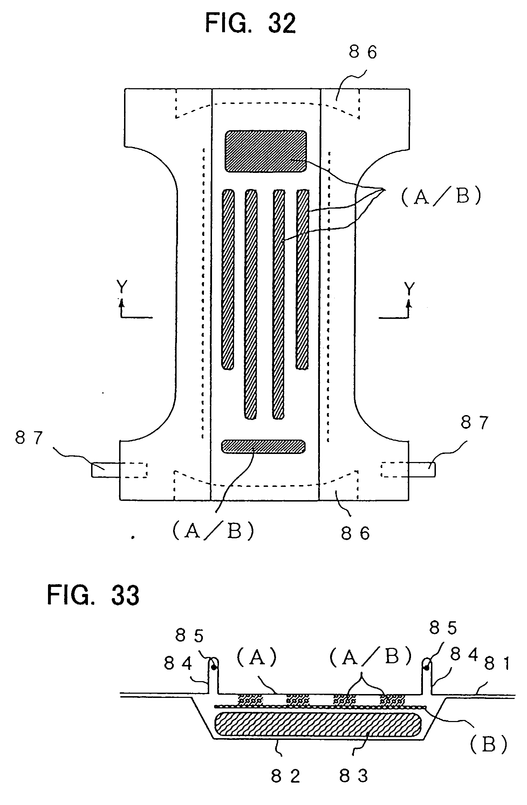

Each of Figures 32 and 33 illustrates diaper to which the water-permeable composite sheet obtained by the above treatment is applied, wherein the reference numeral 82 represents a back sheet which was provided opposite a surface material (81); (83) represents an absorbent; (84) represents a standing-gather which was provided to stand up under the action of an elastic body (85); (86) represents awest-gather; and (87) represents a binding means. Incidentally, the surface material (81) is formed with an identical hydrophobic material from the side area to the center area via the standing-gather (84). The A/B-component layer in the surface material (81) can be, for example, the above-mentioned structure as shown Figure 23 or 24.

For this diaper, an absorption test of 80ccxthree times was carried out. As a result, very speedy water-permeation and absorption were observed from the sintered A/B-component layer, wherein even after absorbing water three times, the A-component layer and the B-component layer were not separated, and furthermore, rewetting at an absorbing area were 0.2cc or less for the first time, and 1cc or less for the second times, and 1cc or less for the third times, which were excellent.

### (Example 13)

### Application to Food Packaging Material:

A hydrophobic A-component layer and a hydrophilic R-component I aye r which are the same layers as ones used in Example I were laid on top of each other, and pa i r of chromium-plated rollers having flat surfaces, which had been heated to a temperature of 140°C under a pressure of a gage pressure of 3kg/cm² and thereby said layers were heated and pressed from both sides of the A-component layer and t he B-component layer.

Thereby, a paper-like sheet whose surface was deformed into a film-shape was obtained. The A-component layer and the B-component layer of this sheet were almost completely fused, and had a stable water-permeability, while a back flow of a liquid was little observed. Furthermore, the sheet was excellent in surface-strength and lint-resistance property, and showed lint-resistance property of top-grade according to a test with cellophane tapes.

A PE-film having a thickness of 20/ µm was laminated to the side of the B-component layer of the above sheet which was sintered all over the surface so as to form a three-layer body. This body was used in order to form a pouch having a gusset, which had a volume of about 200cc and was opened at one side only. A frozen Chinese bun was put into this pouch and the opening was sealed, and then was heated in a common household electronic oven for a period of thirty minutes. The pouch was left for a period of twenty minutes as it was, and then opened. Consequently, neither adhesion of water-drops on the surface of the content nor a cling-state thereon was observed, and a soft and full state had been maintained.

### (Example 14)

### Application to Sanitary Materials

### Preparation of Hydrophobic A-Component Layer

### A hydrophobic PP-spunbond nonwoven fabric (18g/m²) prepared.

### Preparation of Hydrophilic B-Component Layer

A homogeneously mixed card-web of 30g/m² comprising 50% of a bicomponent fiber (trade name "ES Fiber" made by Chisso Co., LTD.) of 2dx5lmm and 50% of a polyester fabric (trade name "BACTEKILLER" made by KANEBO, LTD.) of 3dx5lmm was prepared, wherein said bicomponent fiber comprised PE/PP and was that of a sheath/core type; and said polyester was made to be hydrophilic by treating with a surface active agent and contained silver and zeolite.

### Sintering-Treatment of A-Component Layer and B-Component Layer

The above B-component layer was laid on top of the above A-component layer, and passed through pair-rollers comprising a combination of a paper roller and a chromium plated roller at a rate of 10m/min. while both layers were pressed under a gage pressure of 2kg/m² so that the A-component layer could make contact with the heated roller, wherein the chromium plated roller had been heated to a surface temperature of 120°C. Thereby, both layers were sintered together.

As a result, the surface of the A-component layer was deformed into a film, while the surface of the B-component layer had a bulky structure with a rough nap, so that water was very speedily absorbed thereby. Furthermore. this sheet showed a remarkably excellent antibacterial property in an antibacterial test according to Shake flask method. Utilization as Surface Material of Diaper.

A top sheet of a diaper a sheet of the present working example was incorporated into an adult diaper having the same structure as that of a commercial one, while the sheet was arranged so that the surface of the A-component layer could serve as a surface to make contact with a body, so as to carry out an absorbing test about this diaper. The results of a three-times speed test which was carried out at 30-minute intervals were as follows:

| | | |
|---|---|---|
| First Time | 1OOcc | 25sec |
| Second Time | 1OOcc | 33sec |
| Third Time | 1OOcc | 40sec |

Incidentally, a rewet value after third absorption was 1.2g/100cm² which showed a remarkably excellent performance.

### Utilization as Cushion Material of Diaper

In a baby diaper of M-size having the common structure that an absorbent is arranged inside the top sheet of PP-spunbond, the sheet of the present working example was inserted between the top sheet and the absorbent so that the B-component layer can make contact with the top sheet, and the A-component layer can make contact with the absorbent, and an absorption test about this sample was carried out. Incidentally, the blank was a common one with no sheet of the present working example.

**Table 5**

| Absorbing Speed (sec) | Sample | Blank |
|---|---|---|
| First Time (5Occ) | 18 | 28 |
| Second Time(5Occ) | 25 | 38 |
| Third Time (5Occ) | 32 | 45 |
| Rewetting (g/1OOcm²) | 1.6 | 2.6 |

From the above results, it is apparent that according to the incorporation of a sheet in the present working example, absorbing speed is remarkably increased. and rewetting is controlled.

### (Example 15)

### Application to Blanket Cover and Sheets Used in Hospital:

### Preparation of Hydrophobic A-Component Layer

As a sheet material for an A-component layer. a card web having a specific weight of 15g/m² was prepared from a blended fiber comprising 50% of a hydrophobic PP/PE-bicomponent fiber staple (trade name "ES FIBER" made by Chisso Co., Ltd.) of 2dx45mm, in which a spinning oil solution had been used in an amount as small as possible, and 50% of a PET fiber of 1.5dx45mm.

### Preparation of Hydrophilic B-Component Layer

A card web having a specific weight of 20g/m² was prepared from a blended fiber comprising 30% of the same PP/PE-bicomponent fiber staple as the one used as the A-component layer and 70% of a rayon fiber (trade name KITOPOLY) made by Fujibosekl Co., Ltd.) of 1.2dx35mm.

Then, the above A-component layer and the B-component layer were laid on top of each other, and were passed through a hot air conveyer at a temperature 130°C, so that the A-component layer and the B-component layer were thermo-united with each other.

### Sintering-Treatment of A-Component Layer and B-Component Layer

The above thermo-united sheet was passed through rollers so that the surface of the A-component layer could make contact with a heating roller which had a diameter if 50cm and a flat surface, so that the sheet was sintered. The heating roller had a surface temperature of 140°C, and the passing speed was 2sec/meter.

According to this sintering treatment. the surface of the A-component layer was deformed into a film, while the whole were flat and soft, and very little had a rough nap and the falling-of f of lint. Furthermore, this sheet was excellent in water-absorbing property, and in antibacterial property particularly MRSA resistant property. A test in which the above water permeable composite sheet was used for twenty patients as sheets and blanket covers used in a hospital, was carried out. There were no problems such as a rough nap during the test, and the decision that the touch was excel lent was obtained. Furthermore, no MRSA-infected persons were caused in the patient s during the test.

### (Example 16)

### Application to Meat Package and Tray:

### Preparation of Hydrophobic A-Component Layer

A spunbond nonwoven fabric of 20g/m² (trade name "ELVES" made by UNITIKA LTD.) comprising a bicomponent fiber which comprises PET as a core and PE as a sheath. was prepared as a hydrophobic web for an A-component layer.

### Preparation of Hydrophobic B-Component Layer

A mixed card web comprising 60% of a PE/PET-bicomponent fiber of 2dx45mm which was denatured to be hydrophilic, and 40% of a high absorbing fiber (trade name "VEROASIS" made by KANEBO, Ltd.) of 3dx5lmm was treated in a hot air conveyer, so as to prepare a through-air-bond nonwoven fabric of 30g/m².

### Sintering of A-Component Layer and B-Component Layer

The above-mentioned A-component layer and B-component layer were laid together, and passed between a chromium-plated roll heated to have a surface temperature of 150°C and a paper roll at a pressure of 2kg/cm², with the A-component layer facing to the chromium-plated surface, thereby to obtain water permeable composite sheet of which the surface of the A-component layer became a film and the B-component layer side kept the non-woven fabric nature.

The water permeable composite sheet has excellent in water permeability from the top surface to the back surface of the A-component layer, and showed the one-way water permeation caused due to the fact that the liquid moved to the back surface was absorbed by the highly absorbing fibers, thereby to keep the surface of the A-component layer dry.

The above water permeable composite sheet was laid on a food tray of polystyrol with the A-component layer being upper side, and a cut of frozen beef for steak was put thereon, and wrapped with a wrapping film. A defrost operation was conducted by leaving it at a room temperature for 4 hours. No drip and color change on the cut beef was found.

### (Example 17)

### Application to Super Thin Sanitary Napkins

### Preparation of Hydrophobic A-component Layer

As a sheet material for the A-component layer, a three-layer non-woven fabric (trade name "UNICELL" manufactured by TEIJIN, Co.) which comprises both surface layers of PP fibril burst fibers and a core layer of PET filament web, with these layers being integrated by heat and press.

### Preparation of Hydrophilic B-component Layer

For a sheet material for the B-component layer, highly water-absorbent property (trade name "CM modified TECCEL" manufactured by SHINOJI, Co.), which consists of a non-woven fabric obtained by water entanglement of PP spun bond (12g/m²) with wood pulp (80g/m²) and subjected to treatment for carboxymethylation was prepared.

### Sintering of A-component layer and B-component layer

On the above A-component layer the B-component layer was laid with its spun bond surface contacting, and an iron heated at about 140°C was pressed at both sides of the A-component layer and B-component layer. Then, the surface of the A-component layer which became as film was subjected to treatment for causing the surface uneven.

The water permeable composite sheet had a rapid water permeation speed and excellent absorbing property.

On the B-component layer side of the above water permeable composite sheet was positioned a PE film and subjected to a test which is generally conducted to sanitary napkins.

At the test using an artificial blood, the test sample showed rapid absorbing speed and less than 0.1cc of rewet, and good stain free property and a sufficient absorbing capacity with 1mm thick.

Furthermore, for the purpose that the balance between stain free property and porosity is important, it is suitable to use the one that a water permeable composite sheet 90 comprising A/B-component layer formed by sintering over entire surfaces of the A-component layer and B-component layer and having pores 91 to have a solid cross-section. The pores 91 having a diameter less than 1mm act, different from a sheet comprising a single layer PE film having pores, to provide a stable water permeability and prevent moisturization obtained from easy movement of moisture due to air permeability even at the portions where no pore exists, thereby to have good applicability especially for women and children. The pores 91 are provided for avoiding close contact the skin of the human body due to uneven surface, as well as separating the solid component of the body liquid, rather than for expecting liquid permeability and, therefore, the pores 91 are not necessarily penetrating through the sheet, and may be a deep emboss.

### (Example 18)

### Example of a Combination with Absorbent Sheet

### Preparation of Hydrophobic A-component Layer

As an A-component layer, PE/PET bicomponent spunbond non-woven fabric (trade name"ELBES" manufactured by YUNICHIKA, Co.) was prepared.

### Preparation of Hydrophilic B-component Layer

To a polyether type continuous pore urethane foam sheet (foam rate: 40 times, gravity: 25kg/cm³) a powder of upper absorbent polymer (SAP) was sprayed, and a web of 30g/m² consisting of 60% of hydrophilicated 2dx45mm PE/PET fibers (trade name "SOFIT" manufactured by KURARE, Co.) and 40% of 1.5dx45mm viscose rayon fibers was laid thereon. The both ware needle punched to form a absorbent sheet wherein the pores of the urethane foam and the spaces between fibers were filled with SAP.

### Sintering of A-component layer and B-component layer

The above-mentioned A-component layer and B-component layer were laid together, and passed between a stainless roll (heated to have a surface temperature of 120°C) and flat surface chromium-plated roll (not heated) at a pressure of 6kg/cm² gauge, with the A-component layer facing to the stainless roll for sintering.

As a result, the thickness of 8mm at the not treated state was reduced to a quarter or about 2.2mm. In the absorbent sheet thus obtained, the A-component layer and B-component layer were closely joined together by fusion of the PE component contained in the both, and had a high speed water-absorption at the A-component layer side.

### Water-Absorption Test

The sheet of this Example was laid on a vacant water tank with the A-component layer side being upper, and a great deal of water was pored at a stretch for a free absorbing test. The thickness of the sheet increased to about 15mm or 7 times by swelling, but no breaking was observed after pulled up by hand, and showed a configuration maintaining property like as hard jelly.

For dust-free wipes, blood absorbing mat, food wrapping material for electric oven, a water-absorbent property, lint-free property at surface, and less generation of dust from inside is much important than the touch feeling at the surface. In this sense, more suitable water-absorbent composite sheet for such purpose is that the surface is sufficiently heated and pressed to become a film.

For the appliance to sheets, drapes and the like used in hospitals, it is possible to combine an antibacterial material and then to sinter, thereby to give an antibacterial property, and biobarrier property by controlling the porous structure at the surface.

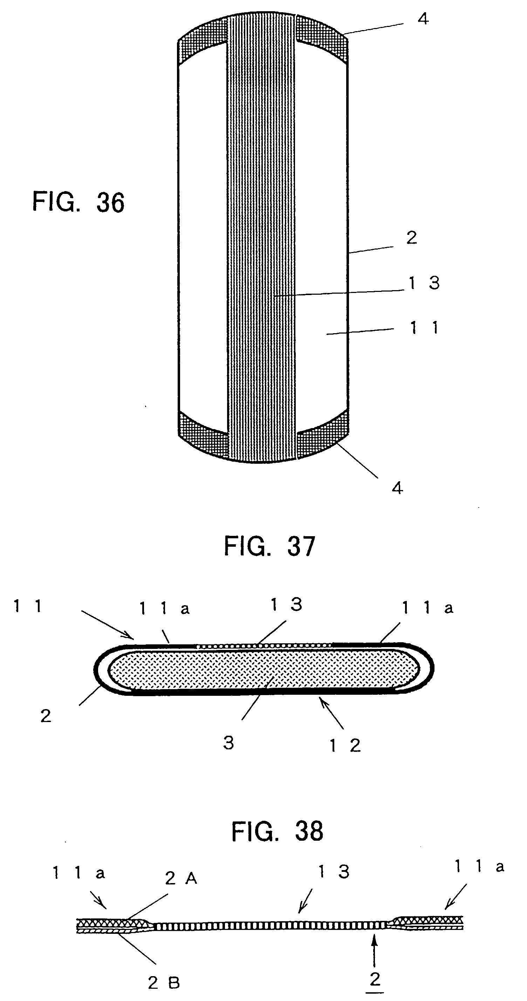

In Figures 36 and 37, the absorbent article in the form of a sanitary napkin comprises a tubular sheet material 2 in which a substantially rectangular absorbent body 3, both ends of the sheet material 2 are sealed at a joining portions 4. The inner absorbent body 3 may be a conventional sanitary napkin as it is.

Sheet material 2 comprises an inner sheet portion 11 facing to the human body when worn, and an outer sheet portion 12 positioned outside, and at a central portion in the width direction of the inner sheet portion 11 is provided a liquid permeable area 13 having about one third of the total width of the sanitary article and extending along the lengthwise. Thus, the inner sheet portion 11 has a liquid permeable property only at the liquid permeable area 13, but the areas 11a disposed at both sides thereof is low liquid permeable. The outer sheet portion 12 consists of liquid impermeable material.

The inner sheet portion 11, as illustrated in section in Figure 38, comprises an A-component layer 2A of hydrophobic material and a B-component layer 2B of hydrophilic material laid one on the other, and the A-component layer and B-component layer are mutually integrated by sintering only at the liquid permeable area 13.

Thus, in the low liquid permeable area 11a the A-component layer and B-component layer exist independently, even if they contact to each other. On the other hand, in the liquid permeable are 13, the A-component layer and B-component layer are mutually integrated in a manner that the A-component layer which is easily fusible is diffused inside of the B-component layer which is more thermally stable but hydrophilic. Therefore, the liquid permeable area 13 positioned at center has a low hydrophilic property but a high liquid permeability.

When such absorbent article is contacted to a liquid, the liquid will permeate through the central liquid permeable area 13 and absorbed by the absorbent body 3 positioned inside, thereby to show an excellent liquid absorbing property. In addition, the surface has a low hydrophilic property at its surface, so that a dry feeling is kept continually.

Furthermore, the low liquid permeable areas 11a are provided at both sides of the liquid permeable area 13, so that it is difficult to occur a side leak owing to reverse current of the liquid once absorbed by the absorbent body 3.

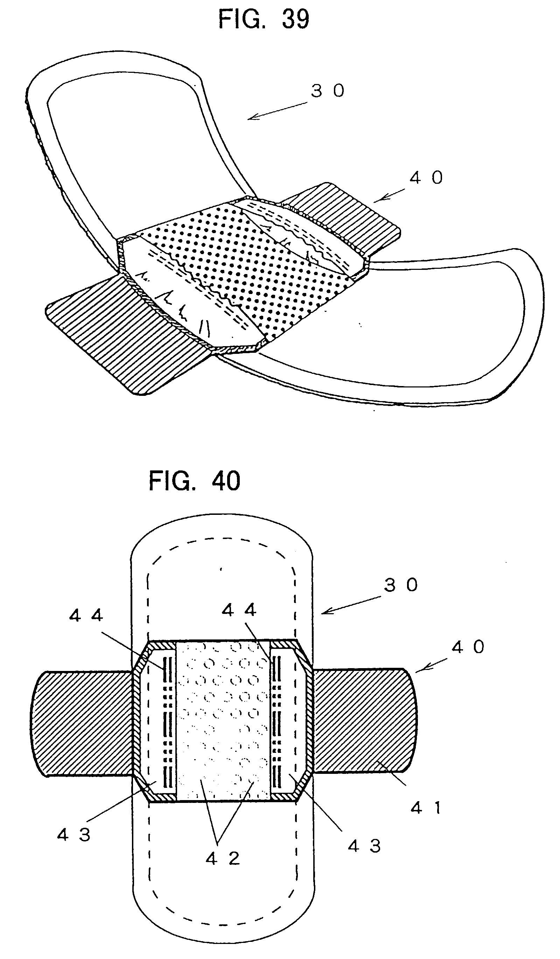

Figures 39 and 40 show another absorbent body. The absorbent body comprises a top sheet of liquid permeable sheet material, a back sheet of liquid impermeable sheet material, an absorbent body 30 having a conventional structure and disposed therebetween, and a liquid controlling unit 40 disposed at approximately central portion along the lengthwise direction of the absorbent body 30.

The controlling unit 40 has a substantially rectangular configuration having a longer length than and substantially equal width of the absorbent body 30, and connected to the absorbent body 30 by a suitable means such as heat seal. Optionally, wings 41 may be provided to extend outside from the side edges.

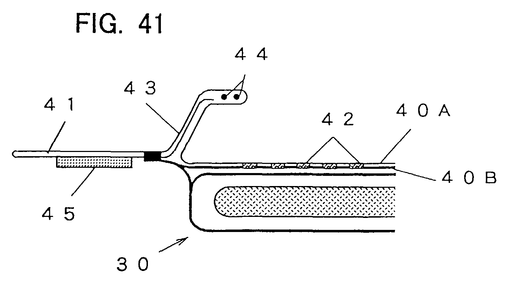

As seen from the cross section shown in Figure 41, the controlling unit 40 comprises, like as the embodiment shown in Figures 39 and 40, an A-component layer of easily fusible material and a B-component layer having a higher heat resistance than A-component layer laid one on the other, and mutually integrated by sintering at a number of round portions 42 to form a liquid permeable area. The sintered liquid permeable portion has a low hydrophilic property but a large liquid permeability as mentioned above.

Part of the A-component layer forms strip-like cuffs 43 extending from both sides, and at inner end of each cuff 43 is provided with an elastic member 44 such as rubber strip. Therefore, the cuffs 43 will stand in the worn state to form a barrier as shown in Figure 38. Numeral 45 depicts a pressure sensitive adhesive layer provided on one surface of the wings 41.

Also, in an absorbent article shown in Figures 39-41, liquid supplied on the controlling unit 40 will penetrates through the sintered liquid permeable area 42 of the liquid permeable area 11 and then is absorbed by the inner absorbent body 30. Even when a larger amount liquid is supplied over the absorbing ability, side leak will be prevented by the side barrier formed by cuffs 43 positioned at both sides of the liquid permeable area 11. In addition, the wings 41 and the cuffs 43 are formed by extension of the A-component layer 40A in which the liquid permeable area 42 is formed, so that these components can be prepared using a smaller kinds of materials compared to the conventional products.

### Industrial Applicability

As has been stated, the porous composite sheet of the present invention comprises a combination of two kinds of materials having a porous structure but having different fusibility, and at least part of the easily fusible material is permeated at the fused state into the spaces of the porous material having high thermal stability, and pressed to fill up and set by cooling.

Therefore, by selecting the kinds of materials, and condition at melting and pressing for filling for sintering, it is possible to obtain a porous composite sheet having various porosity over wide range and desired characteristics.

Especially, a combination of a first layer of hydrophobic material and a second layer of hydrophilic and porous material is optimum for various sanitary articles and medical articles, since the composite sheet has a proper permeability to air or moisture, and excellent draping property.

Furthermore, in the sintered porous composite sheet of the present invention, excellent biobarrier property is obtained by using a water repellent micro fibril layer as a first porous layer and a hydrophilic cellulose layer as a second porous layer. In this case, the composite sheet has a good biobarrier property suitable for surgical appliance such as surgical gowns, surgical drapes, masks and the like. Also, such composite sheet is suitable for filters for electric vacuum cleaners due to its multi-layer micorporous structure and good dust preventing property.

In addition, according to the method of the present invention, it is possible to manufacture the above sintered porous composite sheet easily and in an industrial scale.

## Claims

1. A sintered porous composite sheet, comprising an A/B-component layer in which A-component having easily fusible properties and B-component having relatively higher thermal stability as compared with said A-component are sintered together and coexist.

2. A sintered porous composite sheet, comprising the two layers of: an A/B-component layer essentially consisting of an A-component having easily fusible properties, and a B-component having relatively higher thermal stability as compared, with said A-component being sintered together and coexisting; and said B-component layer having low thermal deformation.

3. A sintered porous composite sheet, characterized by having a structure in which a first porous layer is laid on top of a second porous layer, said first layer comprising A-component which has easily fusible properties, said second layer comprising B-component which has relatively higher thermal stability as compared with A-component, wherein said composite sheet comprises the three layers of: an A-component layer which is molten and resolidified; an A/B-component layer in which A-component and B-component are sintered together and coexist; and a B-component layer having low thermal deformation.

4. A sintered porous composite sheet according to any one of claims 1 to 3, wherein A-component is hydrophilic, and B-component is hydrophobic.

5. A sintered porous composite sheet according to any one of claims 1 to 4, wherein the temperature difference of the fusible temperature of A-component and the melting temperature of said B-component is 30O C or more.

6. A sintered porous composite sheet according to any one or two of claims 3 to 5, wherein 50% or more of said A-component in said first layer is molten and migrated into said A/B-component layer.

7. A sintered porous composite sheet according to any one of claims 3 to 6, whe rein said first layer is a nonwoven fabric sheet comprising melting synthetic fibers.

8. A sintered porous composite sheet of any one of claims 3 to 6, wherein said first layer is a nonwoven fabric sheet comprising easily-melting polyolefine fibers.

9. A sintered porous composite sheet according to any one of claims 3 to 6, wherein said first layer is a nonwoven fabric comprising bicomponent fibers in which an easily fusible polymer is used as a sheath material, and a relatively fusible resistant polymer is used as a core material.

10. A sintered porous composite sheet according to any one of claims 3 to 6, wherein the main component of said first layer is a nonwoven fabric sheet comprising microfibers having a diameter of 0.5 d or less.

11. A sintered porous composite sheet according to any one of claims 3 to 6, wherein said first layer is an easily fusible polyolefine web which is obtained according to Meltblown method; and said second layer is a nonwoven fabric which is obtained according to spunbond method, which comprises bicomponent fibers in which polyethylene is used as a sheath, and polyester is used as a core.

12. A sintered porous composite sheet according to any one of claims 3 to 6, wherein said first layer is a wet-formed web comprising a synthetic polyolefine pulp; and said second layer is a nonwoven fabric which is obtained according to Spunbond method, which comprises bicomponent fibers in which polyethylene is used as a sheath, and polyester is used as a core.

13. A sintered porous composite sheet according to claim 4, wherein said first layer is a hydrophobic nonwoven fabric having easily fusible properties; and said second layer is a hydrophilic cellulose-type porous sheet having thermal stability.

14. A sintered porous composite sheet according to claim 4, wherein said second layer is a water-absorptive nonwoven fabric comprising cellulose fibers.

15. A sintered porous composite sheet according to claim 4, wherein said second layer is a wood-pulp sheet which is formed according to Airlaid method.

16. A sintered porous composite sheet according to claim 4, wherein said second layer comprises a nonwoven-fabric-like web containing high water-absorptive fibers.

17. A sintered porous composite sheet according to any one of claims 3 to 16, wherein said sintered sites are provided merely on a part of the surfaces of said first and second layers.

18. A sintered porous composite sheet according to claim 17, wherein said sintered sites are in the form of a continuous line or a continuous narrow strip.

19. A sintered porous composite sheet according to claim 17, wherein said sintered sites are dispersed in the form of dots.

20. A sintered porous composite sheet according to claim 3 or 4, wherein said first layer is in the form of a corrugated sheet in which crests are formed and mutually in parallel extended, and is continuously or intermittently sintered to said second layer at troughs which are between said crests and mutually adjacent to said crests.

21. A sintered porous composite sheet according to claim 20, wherein said sintered sites are in the form of a continuous line or a successive narrow strip.

22. A sintered porous composite sheet according to claim 20, wherein said sintered sites are in the form of discontinuous lines or discontinuous narrow strips.

23. A sintered porous composite sheet according to any one of claims 2 to 22, wherein another sheet is further jointed to said second layer.

24. A sintered porous composite sheet according to claim 23, wherein said another sheet is a liquid-impermeable sheet.

25. A sintered porous composite sheet according to claim 23, wherein said another sheet is an absorber.

26. A method of producing a sintered porous composite sheet, characterized by the steps of:
mixing a fiber material which A-component having easily fusible properties comprises and a fiber material which B-component having relatively higher thermal stability as compared with said A-component fiber comprises, so as to form a first porous layer in which said fiber material of A-component and said fiber material of B-component are mixed, while a second porous layer is formed, which contains both said fiber material which A-component comprises and said fiber material which B-component comprises, wherein the content of said fiber material which B-component comprises is larger than that of said fiber material which B-component comprises; and
superposing said first layer and said second layer, and thermal treating said first layer and said second layer under such a temperature condition that A-component is hot-molten while B-component is stable and under pressure, and then cooling and solidifying the same, so as to two layer comprising an A/B component layer and a layer which is rich in the content of B-component layer having relatively lower heat-changes.

27. A method of producing a sintered porous composite sheet according to claim 26, wherein said fiber material comprising said A-component and said fiber material comprising said B-component are selected from synthetic fibers, chemical fibers and regenerated fibers, respectively.

28. A method of producing a sintered porous composite sheet according to claim 26 or 27, wherein a mixing ratio(A/B ratio) of a fiber material which A-component in said first layer comprises to another fiber material which B-component in said first layer comprises is 0.5 or more; and A/B ratio in said second layer is 1.0 or less.

29. A method of producing a sintered porous composite sheet, characterized by superposing a first porous layer and a second porous layer, said first layer comprising A-component which has easily fusible properties, said second layer comprising B-component which has relatively higher thermal stability as compared to said A-component; thermal treating said first layer and said second layer under pressure and under such a temperature condition that A-component is hot-molten while B-component is stable, so as to melt A-component and move A-component into B-component; and thereafter cooling and solidifying the same so as to form the three layer of a molten and resolidified A-component layer, A/B component layer, and a B-component layer having low thermal changes.

30. A method of producing a sintered porous composite sheet according to claim 29, wherein said first layer is a polyolefine spunbond nonwoven fabric having easily fusible properties; said second layer is a wood-pulp sheet which is produced by forming a fibers mat from a fibers-dispersed liquid having a wood pulp as a main component according to a wet method and thereafter dewatering and drying the same, wherein said thermal treatment is carried out in the temperature range in which cellulose is not thermally decomposed, and followed by cooling.

31. A method of producing a sintered porous composite sheet according to claim 29, wherein said first layer and said second layer are mutually entangled by a high-pressure stream of water or a needle-punch, and mixed so as to form three layers of a layer consisting of A-component, a mixed layer of A-component and B-component, and a layer consisting of B-component; and said thermal treatment is carried out.

32. A method of producing a sintered porous composite sheet according to claim 29, wherein said first layer is a spunbond nonwoven fabric, and said second layer is a pulp fibers mat; said first layer is subjected to a jet of a high-pressure stream of water in a wetting state so as to slip between spunbond layers and pulp-fibers of said second layer; and said thermal treatment is carried out.

33. A method of producing a sintered porous composite sheet according to claim 29, wherein said first layer is a polyolefine synthetic fiber web having easily melting properties which is obtained according to Card method, and said second layer is a web comprising cellulose fibers, wherein said first layer and said second layer are superposed and mutually slipped with a high-pressure stream of water, and said thermal treatment is carried out and followed by cooling.

34. A Velcro zipper, characterized in that said second layer of a sintered porous composite sheet of claim 2 or 3 is folded by the heat contraction of said first layer thereof, and said second folded porous layer is formed as female sections.

35. A composite absorber, characterized in that said second layer of a sintered porous composite sheet of claim 2 or 3 has pleats which are folded the heat contraction of said first layer, and a powdered polymeric absorbent is stored on the troughs of said pleats.

36. A water-permeable composite sheet, characterized in that said composite sheet comprises a first web-like hydrophobic layer and a second hydrophilic layer, said first layer comprising a hydrophobic material, said second layer comprising a hydrophilic material which is positioned close to said first layer;
said first layer and said second hydrophilic layer contain a common component comprising an easily fusible material; and
said first layer and said second hydrophilic layer are bonded to each other at sintered sites which are formed by sintering said common component all over the surface of each layer or at part thereof,
whereby said composite sheet has water permeability at said sintered sites.

37. A water-permeable composite sheet of claim 36, wherein said common component is a fiber material having easily fusible properties.

38. A water-permeable composite sheet according to claim 33 or 37, wherein said common component is bicomponent fibers which has an easily fusible component as a sheath, and a component having higher thermal-stability as compared with said sheath as a ore.

39. A water-permeable composite sheet according to any one of claims 36 to 38, wherein said first hydrophobic layer and said second hydrophilic layer comprise a fiber material having the same structure as that of each other; and said second hydrophilic layer only is treated to have hydrophilicity.

40. A water-permeable composite sheet according to any one of claims 36 to 39, wherein said composite sheet has openings which are provided at said sintered sites.

41. A water-permeable composite sheet according to any one of claims 36 to 40, wherein said second hydrophilic layer comprises a high water-permeable component, whereby water absorption power is increased.

42. A water-permeable composite sheet according to any one of claims 36 to 41, wherein said second hydrophilic layer comprises an antibacterial component, whereby antibacterial effects are provided.

43. An absorber product which comprises a liquid-impermeable outer-sheet and inner-sheet on the opposite side of a body, and an absorber which is positioned between said outer sheet and said inner sheet, characterized in that said inner-sheet comprises a first hydrophobic porous layer and a second hydrophilic porous layer, said first layer being positioned on the outer side and having easily fusible properties, said second layer being positioned on the inner side; and said absorber product has a liquid-permeable area which is formed by mutually sintering said first hydrophobic layer and said second hydrophilic layer at the center area in a cross direction.

44. An absorber product according to claim 43, characterized in that said liquid-permeable area comprises a plural portions which are mutually discontinuous; and are positioned merely at the center section of said inner sheet.

45. An absorber product which comprises a liquid-impermeable outer-sheet and inner-sheet on the opposite side of a body, and an absorber which is positioned between said outer sheet and said inner sheet, characterized in that said absorber product comprises a liquid-control unit and a liquid-impermeable area, wherein said liquid-control unit is positioned in such a state that it is laid on top of said inner-sheet, and comprises a first hydrophobic porous layer and a second hydrophilic porous layer, said first layer being positioned on the outer-side face and having easily fusible properties, said second layer is positioned on the inner-side face, said liquid-impermeable area being formed by mutually sintering said first hydrophobic layer and said second hydrophilic layer at the center area in a cross direction.

46. An absorber product according to claim 45, wherein said liquid-control unit is jointed to both sides of said liquid-permeable sheet, and has low liquid-permeable cuffs which are extended so as to cover both edges of said liquid-permeable sheet.

47. An absorber product according to claim 45 or 46, wherein said liquid-control unit has wings which are jointed to both sides of said liquid-permeable sheet, respectively.
